(19) Europäisches Patentamt
European Patent Office
Office européen des brevets

(11) EP 2 168 569 A2

(12) DEMANDE DE BREVET EUROPEEN

(43) Date de publication:
31.03.2010 Bulletin 2010/13

(51) Int Cl.:
*A61K 8/81* (2006.01) *A61K 8/894* (2006.01)
*A61K 8/896* (2006.01) *A61Q 1/10* (2006.01)

(21) Numéro de dépôt: 09171686.0

(22) Date de dépôt: 29.09.2009

(84) Etats contractants désignés:
AT BE BG CH CY CZ DE DK EE ES FI FR GB GR HR HU IE IS IT LI LT LU LV MC MK MT NL NO PL PT RO SE SI SK SM TR
Etats d'extension désignés:
AL BA RS

(30) Priorité: 30.09.2008 FR 0856549

(71) Demandeur: L'Oréal
75008 Paris (FR)

(72) Inventeurs:
- Dumousseaux, Christophe
92160, Antony (FR)
- Le Merrer, Carole
92120, Mountrouge (FR)
- Pays, Karl
94410, Saint Maurice (FR)

(74) Mandataire: Goudet, Sylvain
L'Oréal
D.I.P.I.
25-29 Quai Aulagnier
92665 Asnières-sur-Seine Cedex (FR)

# (54) Composition et procédé de maquillage des cils comprenant l'application de 2 compositions

(57) La présente invention a pour objet une composition cosmétique comprenant une phase aqueuse continue, au moins un tensioactif siliconé et au moins un polyélectrolyte réticulé. Elle a également pour objet un procédé cosmétique de maquillage ou de soin non thérapeutique des fibres kératiniques comprenant l'application sur les fibres kératiniques:
- d'une première composition telle que décrite ci-dessus et
- d'une seconde composition comprenant :
- soit une phase aqueuse continue, et au moins un polymère filmogène sous forme de particules en dispersion, ledit polymère étant présent en quantité au moins égale à 5% en matières sèches,
- soit au moins un composé ou un mélange de composés qui, lorsque la composition est portée à une température supérieure ou égale à 40°C, confère à ladite composition un caractère filant dmax supérieur ou égal à 5mm,

ladite seconde composition étant, antérieurement, simultanément, ou postérieurement à son application, portée à une température supérieure ou égale à 40°C.

EP 2 168 569 A2

## Description

La présente invention a pour objet une composition cosmétique de maquillage ou de soin non thérapeutique des fibres kératiniques (telles que les cils, les sourcils, les cheveux), ainsi qu'un procédé comprenant l'application sur les fibres kératiniques de cette composition.

Les compositions mises en oeuvre dans le procédé selon l'invention se présentent en particulier sous la forme d'un produit pour les cils ou mascara.

Par mascara, on entend une composition destinée à être appliquée sur les fibres kératiniques : il peut s'agir d'une composition de maquillage des fibres kératiniques, une base de maquillage des fibres kératiniques ou base-coat, une composition à appliquer sur un mascara, dite encore top-coat, ou bien encore une composition de traitement cosmétique des fibres kératiniques. Le mascara est plus particulièrement destiné aux fibres kératiniques d'êtres humains, mais également aux faux-cils.

Pour obtenir un effet recourbant ou allongeant des cils, il est connu d'utiliser des compositions pouvant être appliquées sous l'action d'une source de chaleur. Très souvent ces compostions contiennent un polymère filmogène qui n'est pas soluble dans l'eau à température ambiante ou à la température de chauffage. Ces compositions ont l'inconvénient d'être difficilement démaquillables avec des produits conventionnels.

De la même manière, les compositions aqueuses comprenant une forte quantité (ie >=5% en matière sèche) de polymère filmogène présent sous forme de particules dans ladite phase aqueuse (et donc insoluble dans l'eau), sont souvent difficiles à démaquiller en raison de la présence du polymère.

Le but de la présente invention est de proposer une composition permettant de faciliter le démaquillage des compositions pouvant être appliquées avec une source de chaleur, ainsi que des compositions comprenant une forte quantité de polymère filmogène présent dans une phase aqueuse sous forme de particules.

Le but de la présente invention est également de proposer un procédé de revêtement des fibres kératiniques permettant l'obtention d'un dépôt sur les cils qui présente une bonne tenue dans le temps, ledit dépôt étant facilement démaquillable.

Le but de la présente invention est également de proposer un procédé de revêtement des fibres kératiniques permettant l'obtention d'un dépôt sur les cils qui présente un bon effet allongeant des cils, notamment sous l'action de la chaleur, et de bonne tenue dans le temps, ledit dépôt étant facilement démaquillable.

Les inventeurs ont découvert que les propriétés décrites ci-dessus peuvent être obtenues en utilisant une première composition comprenant une phase aqueuse continue, au moins un polyélectrolyte réticulé et au moins un tensioactif siliconé. Optionnellement, on peut utiliser en outre une seconde composition pouvant être appliquée sous l'action d'une source de chaleur, ou comprenant une forte quantité de polymère filmogène présent dans une phase aqueuse sous forme de dispersion.

De façon plus précise, l'invention a pour objet une composition comprenant une phase aqueuse continue, au moins un polyélectrolyte réticulé et au moins un tensioactif siliconé.

En effet, l'utilisation d'un polyélectrolyte réticulé et d'un tensioactif siliconé permet d'améliorer le démaquillage de cette composition, mais également d'une deuxième composition appliquée sur ladite composition.

L'invention a également pour objet une composition comprenant une phase aqueuse continue, au moins un tensioactif siliconé non ionique de HLB supérieure ou égale à 8 à 25°C, et au moins un polyélectrolyte réticulé choisi parmi le copolymère acrylamide / acrylamido-2-méthyl propane sulfonate de sodium, le glycolate sodique d'amidon réticulé sous forme de poudre, les polyacrylates de sodium réticulés, les copolymères acide polyacryliques acrylates d'alkyle, les copolymères AMPS/méthacrylates d'alkyle polyoxyéthylénés réticulés, et leurs mélanges, et éventuellement en outre au moins 5% en poids de matières sèches de polymère filmogène sous forme de particules en dispersion par rapport au poids total de ladite composition

L'invention a également pour objet un procédé de maquillage des fibres kératiniques, comprenant l'application sur lesdites fibres d'une composition telle que décrite ci-dessus.

L'invention a également pour objet un kit de maquillage et/ou de soin non thérapeutique des fibres kératiniques, notamment des cils ou des sourcils, comprenant :

- une première composition, notamment de maquillage ou de soin non thérapeutique des fibres kératiniques, comprenant une phase aqueuse continue, au moins un polyélectrolyte réticulé et au moins un tensioactif siliconé, et
- une seconde composition de maquillage et/ou de soin des fibres kératiniques.

L'invention a également pour objet un kit de maquillage et/ou de soin non thérapeutique des fibres kératiniques, notamment des cils ou des sourcils, comprenant :

- une première composition comprenant une phase aqueuse continue, au moins un polyélectrolyte réticulé et au moins un tensioactif siliconé, et

- une seconde composition de maquillage et/ou de soin des fibres kératiniques, ladite composition comprenant préférentiellement une phase aqueuse continue, et au moins un polymère filmogène sous forme de particules en dispersion, ledit polymère étant présent en quantité au moins égale à 5% en matières sèches.
  L'invention a également pour objet, selon un mode particulièrement préféré, un kit de maquillage et/ou de soin non thérapeutique des fibres kératiniques, notamment des cils

ou des sourcils, comprenant :

- une première composition comprenant une phase aqueuse continue, au moins un polyélectrolyte réticulé et au moins un tensioactif siliconé et
- une seconde composition de maquillage et/ou de soin des fibres kératiniques, ladite composition comprenant préférentiellement au moins un composé ou un mélange de composés qui, lorsque la composition est portée à une température supérieure ou égale à 40°C, confère à ladite composition un caractère filant dmax supérieur ou égal à 5 mm, et
- un dispositif pour l'application desdites compositions ; et/ou des moyens de chauffage pour porter, antérieurement, simultanément, ou postérieurement à son application, ladite seconde composition à une température supérieure ou égale à 40°C.

La seconde composition peut être portée à une température supérieure ou égale à 40°C antérieurement, simultanément ou postérieurement à son application sur la première couche de première composition, notamment à l'aide d'un dispositif d'application comprenant des moyens de chauffage, tel qu'une brosse chauffante.
L'invention a également pour objet un procédé cosmétique de maquillage ou de soin non thérapeutique des fibres kératiniques comprenant l'application sur les fibres kératiniques :

- d'une première composition comprenant une phase aqueuse continue, au moins un polyélectrolyte réticulé et au moins un tensioactif siliconé puis
- d'une seconde composition, ladite seconde composition comprenant préférentiellement au moins un composé ou un mélange de composés qui, lorsque la composition est portée à une température supérieure ou égale à 40°C, confère à ladite composition un caractère filant dmax supérieur ou égal à 5 mm,

ladite seconde composition étant, antérieurement, simultanément, ou postérieurement à son application, portée à une température supérieure ou égale à 40°C.
Le caractère filant représente l'aptitude de la composition, une fois soumise à une source de chaleur, à former sur les fibres kératiniques, des fils qui, après étirement à l'aide d'un applicateur, sont suffisamment consistants et conservent leur forme. L'utilisation de la chaleur de manière à porter la composition à une température supérieure ou égale à 40°C permet de maîtriser la longueur des fils formés dans le prolongement des cils.
En particulier, après application de la composition ramollie et étirement des fils, ceux-ci se solidifient à température ambiante dans le prolongement de chaque cil et permettent d'obtenir un effet allongeant remarquable.
L'application préalablement à la seconde composition, d'une première composition comprenant une phase aqueuse continue, au moins un polyélectrolyte réticulé et au moins un tensioactif siliconé, permet de former un premier film sur les cils qui résiste à la chaleur lorsque la seconde composition est soumise à une source de chaleur. On obtient sur les cils un premier film (film de base) moins cohésif que le deuxième film formé par la seconde composition au dessus du film de base, ce qui facilite le démaquillage de l'ensemble du dépôt sur les cils.
La seconde composition peut être notamment utilisée en association avec un instrument chauffant, tel qu'une brosse chauffante, qui peut être appliquée sur les cils avant, pendant
ou après que ceux-ci soient revêtus de la composition ou conditionnée dans un dispositif permettant d'appliquer la composition à chaud.
La seconde composition peut être appliquée sur la totalité ou sur l'extrémité supérieure des fibres kératiniques, en particulier des cils. Selon un mode de réalisation, la seconde composition est appliquée sur l'extrémité supérieure des cils.
La première composition est de préférence appliquée sur la totalité du cil.
Les première et seconde compositions comprennent un milieu physiologiquement acceptable, c'est-à-dire un milieu non toxique et susceptible d'être appliqué sur les fibres kératiniques, telles que les cils, les sourcils et les cheveux d'êtres humains, notamment compatible avec la zone oculaire.
La première composition peut être colorée (c'est à dire comprendre au moins une matière colorante telle que définie plus loin) ou non colorée.
Selon un mode de réalisation, la seconde composition cosmétique est colorée, de manière à former des fils colorés au bout des cils.

Selon un autre mode de réalisation, le procédé selon l'invention consiste à :

- appliquer une première composition cosmétique non colorée sur les fibres kératiniques, ladite première composition comprenant une phase aqueuse continue, au moins un polyélectrolyte réticulé et au moins un tensioactif siliconé ; puis
- appliquer une seconde composition cosmétique non colorée sur les fibres kératiniques, ladite seconde composition comprenant de préférence au moins un composé ou un mélange de composés qui, lorsque la composition est portée à une température supérieure ou égale à 40˚C, confère à ladite composition un caractère filant dmax supérieur ou égal à 5 mm,
- porter ladite seconde composition, antérieurement, simultanément, ou postérieurement à son application, à une température supérieure ou égale à 40˚C de manière à former des fils incolores au bout des cils,
- puis après refroidissement des fils, à maquiller les fibres kératiniques et les fils incolores figés dans leur prolongement avec une troisième composition comprenant une ou des matières colorantes, de manière à colorer les fils.

En particulier, la seconde composition comprenant au moins un composé ou un mélange de composés qui, lorsque la composition est portée à une température supérieure ou égale à 40˚C, confère à ladite composition un caractère filant dmax supérieur ou égal à 5 mm, est appliquée sur l'extrémité supérieure des fibres kératiniques, en particulier des cils.

## I) Première composition

Selon un mode de réalisation, la première composition comprend une phase aqueuse continue, au moins un polyélectrolyte réticulé et au moins un tensioactif siliconé.

### a) Phase aqueuse continue

La première composition selon l'invention comprend une phase aqueuse continue.
Par composition à phase aqueuse « continue », on entend que la composition présente une conductivité, mesurée à 25˚C, supérieure à 23 μS/cm (microSiemens/cm), la conductivité étant mesurée par exemple à l'aide d'un conductimètre MPC227 de Mettler Toledo et d'une cellule de mesure de conductivité Inlab730. La cellule de mesure est immergée dans la composition, de façon à éliminer les bulles d'air susceptibles de se former entre les 2 électrodes de la cellule. La lecture de la conductivité est faite dès que la valeur du conductimètre est stabilisée. Une moyenne est réalisée sur au moins 3 mesures successives.
La phase aqueuse peut être constituée essentiellement d'eau ; elle peut également comprendre un mélange d'eau et de solvant miscible à l'eau (miscibilité dans l'eau supérieure à 50 % en poids à 25 ˚C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.
La phase aqueuse (eau et éventuellement le solvant miscible à l'eau) peut être présente, en une teneur allant de 30 % à 98 % en poids, par rapport au poids total de la composition la comprenant, de préférence allant de 40 % à 95 % en poids, et préférentiellement allant de 40 % à 90 % en poids.

### b) Tensioactif siliconé

On entend par « tensioactif siliconé », un composé siliconé comportant au moins une chaîne oxyalkylénée, notamment comportant au moins une chaîne oxyéthylénée (-OCH2CH2-) et/ou oxypropylénée (-OCH2CH2CH2-).
Selon l'invention, on utilise généralement un émulsionnant siliconé choisi de manière appropriée pour l'obtention d'une émulsion huile-dans-eau. En particulier, on peut utiliser un émulsionnant siliconé possédant à 25 ˚C une balance HLB (hydrophile-lipophile balance) au sens de GRIFFIN, supérieure ou égale à 8.
La valeur HLB selon GRIFFIN est définie dans J. Soc. Cosm. Chem. 1954 (volume 5), pages 249-256.
Ces agents tensioactifs siliconés peuvent être choisis parmi des agents tensioactifs siliconés non ioniques, anioniques, cationiques, amphotériques. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.
Les tensioactifs siliconés utilisés préférentiellement dans les compositions selon l'invention sont choisis parmi :

a) les agents tensioactifs siliconés non ioniques de HLB supérieur ou égal à 8 à 25˚C, utilisés seuls ou en mélange;

on peut citer notamment :

les polydiméthylsiloxanes comportant à la fois des groupements oxyéthylénés et des groupements oxypropylénés. On peut citer par exemple le polydiméthylsiloxane à terminaison oxyéthyléne/oxypropylène commercialisé en mélange avec des triglycérides d'acides caprylique/caprique sous la dénomination Abil Care 85 par la société Goldschmidt (nom INCI : BIS-PEG/PPG-16/16 PEG/PPG-16/16 Dimethicone / Caprylic/Capric Triglyceride), le polydiméthylsiloxane à groupement alpha-omega polyéther (OE / OP: 40 / 60), commercialisé sous la dénomination Abil B8832 par la société Goldschmidt (nom INCI : BIS-PEG/PPG-20/20 Dimethicone), le polydiméthylsiloxane oxyéthyléné oxypropyléné commercialisé sous la dénomination Abil B88184 par la société Goldschmidt (nom INCI : PEG/PPG-20/6 Dimethicone)
la diméthicone copolyol de nom INCI PEG/PPG-17/18 DIMETHICONE, telle que celle vendue sous la dénomination Q2-5220 Resin Modifier® par la société DOW CORNING,
la diméthicone copolyol benzoate (FINSOLV SLB 101® et 201® de la société FINTEX),
et leurs mélanges.

b) les tensioactifs siliconés amphotériques tels que les diméthicone copolyols phosphates tels que celui vendu sous la dénomination PECOSIL PS 100® par la société PHOENIX CHEMICAL
c) et leurs mélanges.

Selon un mode préféré, le tensioactif siliconé est choisi parmi les polydiméthylsiloxanes comportant à la fois des groupements oxyéthylénés et des groupements oxypropylénés, la dimethicone copolyol, et leurs mélanges.
De préférence encore, le tensioactif siliconé est choisi parmi les polydiméthylsiloxanes comportant à la fois des groupements oxyéthylénés et des groupements oxypropylénés.
Selon un mode préféré de réalisation de l'invention, le tensioactif siliconé est l'Abil Care 85 ou le Q2-5220 Resin Modifier®.
Le tensioactif siliconé peut être présent en une teneur allant de 0,2 à 20% en poids par rapport au poids total de la première composition, de préférence de 0,5 à 15% en poids et mieux de 1 à 10% en poids.
Selon un mode particulier, le tensioactif siliconé est présent en une teneur d'au moins 0,2% en poids, de préférence d'au moins 5% en poids par rapport au poids total de ladite composition. En particulier, le tensioactif siliconé est présent en une teneur allant de 0,2 à 20% en poids, de préférence de 0,5 à 15% en poids par rapport au poids total de ladite composition.

c) <u>Polyélectrolyte réticulé</u>

Par "polyélectrolyte", on entend une substance macromoléculaire, qui a la faculté de se dissocier, lorsqu'elle est dissoute dans l'eau ou dans tout autre milieu ionisant, pour donner au moins un ion. Autrement dit, un polyélectrolyte est un polymère comportant au moins un monomère ionisable.
En particulier, le polyélectrolyte peut donner des polyions, par exemple des polyanions, lorsqu'il est dissocié dans l'eau. Un polyélectrolyte peut être un polyacide, une polybase, un polysel ou polyampholyte. Dans le cadre de l'invention, il est de préférence un polyacide, et de préférence un polyacide fort.
De façon préférée, le polyélectrolyte réticulé compris dans la première composition cosmétique selon la présente invention est un polymère anionique réticulé.
De préférence, le polyélectrolyte réticulé est en outre apte à former un gel en solution à 0,5 % en poids (en matière sèche).
Les contre-ions des polyions formés lors de la dissociation peuvent être de toute nature, inorganique ou organique.
En particulier, lorsque le polyélectrolyte réticulé est un polymère anionique réticulé, les cations peuvent être des cations alcalins ou alcalino-terreux tels que le sodium ou le potassium ou encore l'ion ammonium.
Le cation sodium $Na^+$ est préféré, c'est pourquoi il est principalement cité dans la liste de polyélectrolytes réticulés qui va suivre, sans que cela constitue une quelconque limitation à ce contre-ion spécifique.
A titre de polyélectrolyte réticulé, on peut citer:

- le copolymère acrylamide / acide 2-méthyl 2-[(1-oxo 2-propényl)amino] 1-propane sulfonique (AMPS) partiellement ou totalement salifié, notamment sous forme de sel de sodium tel que le Simulgel 600® sous forme d'émulsion contenant du polysorbate 80 à titre de tensioactif et contenant de l'isohexadécane à titre de phase huile, vendu par la société SEPPIC, ou encore le Simulgel EG®, le Simulgel A® et le Simulgel 501® vendus par la même société.
Le Simulgel 600® est notamment décrit dans le document FR 2 785 801. Il s'agit en réalité d'un latex inverse. Le polyélectrolyte AMPS est de l'acide 2-méthyl 2-[(1-oxo 2-propényl)amino] 1-propanesulfonique partiellement ou totalement salifié notamment sous forme de sel de sodium ou de sel d'ammonium compris à hauteur de 30 à 50 % en proportions molaires dans le mélange comprenant de l'AMPS ainsi qu'un acrylamide, contenu quant à lui à

hauteur de 50 à 70 %.

- le glycolate sodique d'amidon réticulé sous forme de poudre,
- les polyacrylates de sodium réticulés tel que le Norsocryl S35® vendu par la société ATOFINA, ou le Cosmedia SP® vendu par la société COGNIS,
- les copolymères acide polyacryliques acrylates d'alkyle de type PEMULEN®,
- l'AMPS (Acide polyacrylamidométhyl propane sulfonique neutralisé partiellement à l'ammoniaque et hautement réticulé) commercialisé par la société CLARIANT,
- les copolymères AMPS/méthacrylates d'alkyle polyoxyéthylénés réticulés,
- leurs mélanges.

Conviennent tout particulièrement à l'invention le polyacrylate de sodium réticulé, le copolymère acrylamide/AMPS et leurs copolymères.

Il sera bien entendu fait en sorte que la teneur en polyélectrolyte réticulé soit ajustée de telle manière que l'aptitude au démaquillage soit effectivement améliorée tout en étant non préjudiciable à la tenue à l'eau de la composition cosmétique.

Il est entendu que la quantité en polyélectrolyte réticulé est susceptible de varier significativement selon la nature du polyélectrolyte réticulé. De manière générale, cette quantité est au moins égale à la quantité nécessaire et suffisante pour conférer à ladite composition une meilleure aptitude au démaquillage. Elle est encore qualifiée de quantité efficace. Cette aptitude au démaquillage peut notamment être appréciée à l'aide du test figurant en exemple 1.

Sans que cela constitue une quelconque limitation à l'invention, les inventeurs ont émis l'hypothèse que le polyélectrolyte réticulé joue le rôle de « pompe à eau ». Ainsi, ce rôle de « pompe à eau » apparaît plus nettement lorsque la composition est mise en contact avec une phase aqueuse au moment du démaquillage. Lors du démaquillage, le film de la composition cosmétique selon l'invention se fragilise en surface et entraîne sa rupture mécanique ; il y a alors morcellement du film.

De préférence, le polyélectrolyte réticulé est présent en une teneur (en matières sèches) allant de 0,2 à 15% en poids par rapport au poids total de la première composition, de préférence de 0,5 à 10%, mieux de 0,6 à 5% en poids.

Selon un mode de réalisation, la première composition comprend au moins un polymère filmogène dispersé sous forme de particules dans la phase aqueuse, ledit polymère étant présent en quantité au moins égale à 5% en matières sèches.

Dans ce cas, la première composition peut être utilisée sans deuxième composition. Elle se démaquille facilement avec un démaquillant classique.

d) Polymère filmogène dispersé sous forme de particules dans la phase aqueuse

Dans la présente invention, on entend par « polymère filmogène », un polymère apte à former à lui seul ou en présence d'un agent auxiliaire de filmification, un film macroscopiquement continu et adhérent sur les fibres kératiniques.

Le polymère filmogène est présent dans la composition selon l'invention en une teneur en matières sèches (ou matières actives) au moins égale à 5% en poids par rapport au poids total de la première composition, de préférence allant de 5% à 40 % en poids, mieux de 7 à 30% en poids, et mieux de 10% à 25% en poids.

Le polymère filmogène, lorsqu'il est présent, est dispersé dans la composition sous la forme de particules dans la phase aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90®, Neocryl A-1070®, Neocryl A-1090®, Neocryl BT-62®, Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer, Allianz OPT par la société ROHM & HAAS, les dispersions aqueuses de polymères acryliques ou styrène/acrylique vendues sous le nom de marque JONCRYL® par la société JOHNSON POLYMER ou encore les dispersions aqueuses de polyuréthane vendues sous les dénominations Neorez R-981® et Neorez R-974® par la société AVECIA-NEORESINS, les Avalure UR-405®, Avalure UR-410®, Avalure UR-425®, Avalure UR-450®, Sancure 875®, Sancure 861®, Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.

Selon un mode de réalisation avantageux, la composition selon l'invention comprend au moins un polymère filmogène acrylique sous forme de particules solides en dispersion dans la phase aqueuse, ledit polymère résultant de préférence de la polymérisation d'au moins un monomère à insaturation éthylénique choisis parmi les acides carboxyliques $\alpha,\beta$ -éthyléniques, leurs esters et leurs amides.

Comme acide carboxylique insaturé $\alpha,\beta$-éthyléniques, on peut utiliser l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de ces acides carboxyliques peuvent être choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Il est possible bien entendu d'employer un mélange de ces monomères.

Les esters de l'acide (méth)acrylique particulièrement préférés sont les (méth)acrylates d'alkyle.

Le groupement alkyle des esters peut être soit fluoré, soit perfluoré, c'est-à-dire qu'une partie ou la totalité des atomes d'hydrogène du groupement alkyle sont substitués par des atomes de fluor.

Comme amides desdits acides caboxyliques, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Le polymère filmogène acrylique utilisable selon l'invention peut comprendre, en plus des monomères cités précédement, au moins un monomère styrénique, tel que le styrène ou l'alpha-méthyl styrène.

Comme polymère acrylique, on peut utiliser ceux vendus sous les dénominations "SYNTRAN® 5190 », « SYNTRAN® 5760 », « SYNTRAN ®5009» par la société INERPOLYMER, « DOW LATEX 424® » par la société DOW CHEMICAL.

La première composition peut également comprendre des composés additionnels choisis parmi les sels, les gélifiants hydrosolubles, les cires, les pâteux, les tensioactifs non siliconés et les matières colorantes.

e) <u>Composés additionnels</u>

*<u>Sels</u>*

La première composition selon l'invention comprend de préférence au moins un sel.

Sans que cela constitue une quelconque limitation à l'invention, les inventeurs ont émis l'hypothèse que le sel permet de limiter le gonflement du polyélectrolyte réticulé dans la composition.

Comme sels utilisables, on peut citer notamment les sels des métaux mono-, di- ou trivalents, et plus particulièrement les sels de métal alcalino-terreux et en particulier les sels de baryum, de calcium et de strontium, les sels de métal alcalin et par exemple les sels de sodium et de potassium, ainsi que les sels de magnésium, de béryllium, d'yttrium, de lanthane, de cérium, de praséodyme, de néodyme, de prométhium, de samarium, d'europium, de gadolinium, de terbium, de dysprosium, d'holmium, d'erbium, de thulium, d'ytterbium, de lutétium, de lithium, d'étain, de zinc, de manganèse, de cobalt, de nickel, de fer, de cuivre, de rubidium, d'aluminium, de silicium, de sélénium, et leurs mélanges.

Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'$\alpha$-hydroxyacides (citrates, tartrates, lactates, malates) ou d'acides de fruits, ou encore les sels d'acides aminés (aspartate, arginate, glycocholate, fumarate).

De préférence, on utilise les sels de calcium, de magnésium, de sodium, de potassium et leurs mélanges, et plus particulièrement le chlorure de magnésium, le chlorure de potassium, le chlorure de sodium, le chlorure de calcium, le bromure de magnésium et leurs mélanges.

De préférence, la première composition comprend du chlorure de sodium.

De préférence, le(s) sel(s) est présent en une teneur allant de 0,1 à 5% en poids par rapport au poids total de la première composition, de préférence de 0,5 à 10%, mieux de 0,6 à 5% en poids.

*<u>Epaississants hydrosolubles</u>*

La première composition selon l'invention peut comprendre un épaississant hydrosoluble. Les épaississants hydrosolubles utilisables dans les compositions selon l'invention peuvent être choisis parmi:

les homo- ou copolymères d'acides acrylique ou méthacrylique ou leurs sels et leurs esters et en particulier les produits vendus sous les dénominations VERSICOL F® ou VERSICOL K® par la société ALLIED COLLOID, ULTRAHOLD 8® par la société CIBA-GEIGY,

les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,

les protéines comme les protéines d'origine végétale telles que les protéines de blé, de soja ; les protéines d'origine animale tels que les kératines, par exemples les hydrolysats de kératine et les kératines sulfoniques ;

les polymères de cellulose tels que l'hydroxyéthylcellulose, l'hydroxypropylcellulose, la méthylcellulose, l'éthylhydroxyéthylcellulose, ainsi que les dérivés quaternisés de la cellulose ;

les polymères ou copolymères acryliques, tels que les polyacrylates ou les polyméthacrylates ;

les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;

les polymères d'origine naturelle, éventuellement modifiés, tels que:

les gommes arabiques, la gomme de guar, les dérivés du xanthane, la gomme de karaya ;

les alginates et les carraghénanes ;

les glycoaminoglycanes, l'acide hyaluronique et ses dérivés ;

la résine shellac, la gomme de sandaraque, les dammars, les élémis, les copals ;

l'acide désoxyribonucléïque ;

les muccopolysaccharides tels les chondroïtines sulfate,

et leurs mélanges.

Selon un mode particulier, l'épaississant hydrosoluble est choisi parmi les polymères de cellulose ; de préférence, on utilisera l'hydroxyéthylcellulose.

Le polymère épaississant hydrosoluble peut être présent dans la composition le comprenant en une teneur en matières sèches allant de 0,01 % à 60 % en poids, de préférence de 0,5 % à 40 % en poids, mieux de 1 % à 30 % en poids, voire de 5 à 20 % en poids par rapport au poids total de la composition le comprenant.

*Cires*

La cire considérée dans le cadre de la présente invention est d'une manière générale un composé lipophile, solide à température ambiante (25 °C), déformable ou non, à changement d'état solide/liquide réversible, ayant un point de fusion supérieur ou égal à 30 °C pouvant aller jusqu'à 100 °C et notamment jusqu'à 90 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur ou égal à 45 °C, mieux supérieure ou égale à 50°C, et encore mieux, supérieure ou égale à 60 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :

Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

Les cires pouvant être utilisées dans les compositions selon l'invention présentent généralement une dureté allant de 0,01 MPa à 15 MPa, notamment supérieure à 0,05 MPa et en particulier supérieure à 0,1 MPa.

La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.
Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.
La valeur de la dureté est la force de compression maximale mesurée divisée par la surface du cylindre du texturomètre en contact avec la cire.

A titre illustratif des cires convenant à l'invention, on peut notamment citer les cires hydrocarbonées comme la cire d'abeille, la cire de lanoline, et les cires d'insectes de Chine; la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters, les cires obtenues par hydrogénation catalytique d'huiles animales ou végétales ayant des chaînes grasses, linéaires ou ramifiées, en $C_8$-$C_{32}$-comme l'huile de jojoba hydrogénée, l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S® par la société HETERENE.
On peut encore citer les cires de silicone, les cires fluorées.
On peut également utiliser les cires obtenues par hydrogénation d'huile de ricin estérifiée avec l'alcool cétylique vendues sous les dénominations de Phytowax ricin 16L64® et 22L73® par la société SOPHIM. De telles cires sont décrites dans la demande FR-A-2792190.

On peut utiliser une cire dite cire collante c'est-à-dire possédant un collant supérieur ou égal à 0,7 N.s et une dureté inférieure ou égale à 3,5 MPa.
L'utilisation d'une cire collante peut notamment permettre l'obtention d'une composition cosmétique qui s'applique facilement sur les cils, ayant une bonne accroche sur les cils et qui conduit à la formation d'un maquillage lisse, homogène et épaississant.
La cire collante utilisée peut posséder notamment un collant allant de 0,7 N.s à 30 N.s, en particulier supérieur ou égal à 1 N.s, notamment allant de 1 N.s à 20 N.s, en particulier supérieur ou égal à 2 N.s, notamment allant de 2 N.s à 10 N.s, et en particulier allant de 2 N.s à 5 N.s.
Le collant de la cire est déterminé par la mesure de l'évolution de la force (force de compression ou force d'étirement) en fonction du temps, à 20 °C à l'aide du texturomètre vendu sous la dénomination "TA-TX2i®" par la société RHEO, équipé d'un mobile en polymère acrylique en forme de cône formant un angle de 45°.
Le protocole de mesure est le suivant :
La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.
Le mobile du texturomètre est déplacé à la vitesse de 0,5 mm/s, puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s.
Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.
La cire collante pouvant être utilisée a généralement une dureté inférieure ou égale à 3,5 MPa, en particulier allant de 0,01 MPa à 3,5 MPa, notamment allant de 0,05 MPa à 3 MPa, voire encore allant de 0,1 MPa à 2,5 MPa.
La dureté est mesurée selon le protocole décrit précédemment. Comme cire collante, on peut utiliser un (hydroxystéaryloxy)stéarate d'alkyle en C20-C40 (le groupe alkyle comprenant de 20 à 40 atomes de carbone), seul ou en mélange, en particulier un 12-(12'-hydroxystéaryloxy)stéarate d'alkyle en C20-C40, de formule (II):

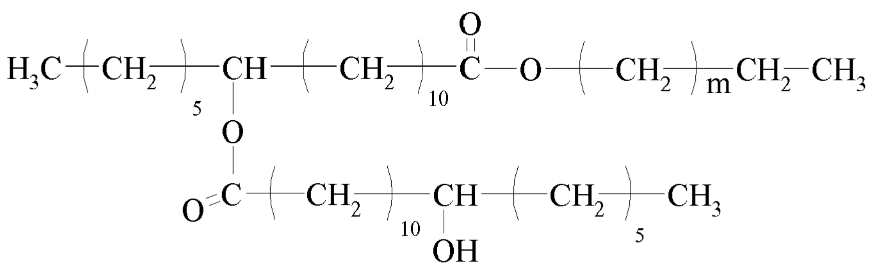

(II)

dans laquelle m est un entier allant de 18 à 38, ou un mélange de composés de formule (II).
Une telle cire est notamment vendue sous les dénominations « Kester Wax K 82 P® », « Hydroxypolyester K 82 P® » et « Kester Wax K 80 P® » par la société KOSTER KEUNEN,

On peut également utiliser des cires fournies sous forme de petites particules ayant une dimension exprimée en diamètre « effectif » moyen en volume D[4,3] de l'ordre de 0,5 à 30 micromètres, en particulier de 1 à 20 micromètres, et plus particulièrement de 5 à 10 micromètres, désignées par la suite par l'expression « micro cires ».

Les tailles de particules peuvent être mesurées par différentes techniques, on peut citer en particulier les techniques de diffusion de la lumière (dynamiques et statiques), les méthodes par compteur Coulter, les mesures par vitesse de sédimentation (reliée à la taille via la loi de Stokes) et la microscopie. Ces techniques permettent de mesurer un diamètre de particules et pour certaines d'entre elles une distribution granulométrique.
Comme micro cires pouvant être utilisées dans les compositions selon l'invention, on peut citer notamment les micro cires de carnauba telles que celle commercialisée sous la dénomination de MicroCare 350® par la société MICRO POWDERS, les micro cires de cire synthétique telles que celle commercialisée sous la dénomination de MicroEase 114S® par la société MICRO POWDERS, les micro cires constituées d'un mélange de cire de carnauba et de cire de polyéthylène telles que celles commercialisées sous les dénominations de Micro Care 300® et 310® par la société MICRO POWDERS, les micro cires constituées d'un mélange de cire de carnauba et de cire synthétique telles que celle commercialisée sous la dénomination Micro Care 325® par la société MICRO POWDERS, les micro cires de polyéthylène telles que celles commercialisées sous les dénominations de Micropoly 200®, 220®, 220L® et 250S® par la société MICRO POWDERS et les micro cires de polytétrafluoroéthylène telles que celles commercialisées sous les dénominations de Microslip 519® et 519 L® par la société MICRO POWDERS.

La cire peut représenter de 1 à 50% en poids par rapport au poids total de la première composition, de préférence de 2 à 40% en poids et mieux de 5 à 20% en poids.

*Pâteux*

Par "composé pâteux" au sens de la présente invention, on entend un composé gras lipophile à changement d'état solide/liquide réversible et comportant à la température de 23°C une fraction liquide et une fraction solide.
Un composé pâteux est à la température de 23°C, sous la forme d'une fraction liquide et d'une fraction solide. En d'autres termes, la température de fusion commençante du composé pâteux est inférieure à 23°C. La fraction liquide du composé pâteux, mesurée à 23°C, représente de 20 à 97% en poids du composé pâteux. Cette fraction liquide à 23°C représente plus préférentiellement de 25 à 85%, et mieux de 30 à 60% en poids du composé pâteux.
La fraction liquide en poids du composé pâteux à 23°C est égale au rapport de l'enthalpie de fusion consommée à 23°C sur l'enthalpie de fusion du composé pâteux.

L'enthalpie de fusion consommée à 23°C est la quantité d'énergie absorbée par l'échantillon pour passer de l'état solide à l'état qu'il présente à 23°C constitué d'une fraction liquide et d'une fraction solide.
L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide. Le composé pâteux est dit à l'état liquide lorsque l'intégralité de sa masse est sous forme liquide.
L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999.

L'enthalpie de fusion du composé pâteux est la quantité d'énergie nécessaire pour faire passer le composé de l'état solide à l'état liquide. Elle est exprimée en J/g.

La fraction liquide du composé pâteux, mesurée à 32°C, représente de préférence de 40 à 100% en poids du composé pâteux, mieux encore de 50 à 100% en poids du composé pâteux. Lorsque la fraction liquide du composé pâteux mesurée à 32°C est égale à 100%, la température de la fin de la plage de fusion du composé pâteux est inférieure ou égale à 32°C.

La fraction liquide du composé pâteux, mesurée à 32˚C, est égale au rapport de l'enthalpie de fusion consommée à 32˚C sur l'enthalpie de fusion du composé pâteux. L'enthalpie de fusion consommée à 32˚C est calculée de la même façon que l'enthalpie de fusion consommée à 23˚C.

Le composé pâteux a de préférence une dureté à 20˚C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20˚C au centre de 5 échantillons. Le cylindre est introduit dans chaque échantillon, la profondeur de pénétration étant de 0,3 mm. La valeur relevée de la dureté est celle du pic maximum.

Le composé pâteux peut être choisi parmi les composés synthétiques et les composés d'origine végétale. Un composé pâteux peut être obtenu par synthèse à partir de produits de départ d'origine végétale.

Le composé pâteux est avantageusement choisi parmi:

la lanoline et ses dérivés tels que l'alcool de lanoline, les lanolines oxyéthylénées, la lanoline acétylée, les esters de lanoline tels que le lanolate d'isopropyle, les lanolines oxypropylénées,
les composés siliconés polymères ou non-polymères comme les polydiméthysiloxanes de masses moléculaires élevées, les polydiméthysiloxanes à chaînes latérales du type alkyle ou alcoxy ayant de 8 à 24 atomes de carbone, notamment les stéaryl diméthicones,
les composés fluorés polymères ou non-polymères,
les polymères vinyliques, notamment

- les homopolymères d'oléfines,
- les copolymères d'oléfines,
- les homopolymères et copolymères de diènes hydrogénés,
- les oligomères linéaires ou ramifiés, homo ou copolymères de (méth)acrylates d'alkyle ayant de préférence un groupement alkyle en $C_8$-$C_{30}$
- les oligomères homo et copolymères d'esters vinyliques ayant des groupements alkyles en $C_8$-$C_{30}$,
- les oligomères homo et copolymères de vinyléthers ayant des groupements alkyles en $C_8$-$C_{30}$,

les polyéthers liposolubles résultant de la polyéthérification entre un ou plusieurs diols en $C_2$-$C_{100}$, de préférence en $C_2$-$C_{50}$,
les esters et les polyesters,
et leurs mélanges.

Le composé pâteux peut être un polymère, notamment hydrocarboné.

Un composé pâteux siliconé et fluoré préféré est le polyméthyltrifluoropropyl-méthylalkyl-diméthylsiloxane, fabriqué sous la dénomination X22-1088 par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

Parmi les polyéthers liposolubles, on peut notamment citer les copolymères d'oxyde d'éthylène et/ou d'oxyde de propylène avec des oxydes d'alkylène en $C_6$-$C_{30}$. De préférence, le rapport pondéral de l'oxyde d'éthylène et/ou de l'oxyde de propylène avec les oxydes d'alkylène dans le copolymère est de 5:95 à 70:30. Dans cette famille, on citera notamment les copolymères blocs comprenant des blocs d'oxydes d'alkylène en $C_6$-$C_{30}$ ayant un poids moléculaire allant de 1 000 à 10 000, par exemple un copolymère bloc polyoxyéthylène/polydodécylène glycol tel que les éthers de dodécanediol (22 mol) et de polyéthylène glycol (45 motifs oxyéthylène ou OE) commercialisés sous la marque ELFACOS ST9 par Akzo Nobel.

Parmi les esters, on préfère notamment :

- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras comme l'acide stéarique, l'acide caprique, l'acide stéarique, l'acide isostéarique et l'acide 12-hydroxystéarique, tels que ceux notamment commercialisés sous la marque Softisan 649 par la société Sasol ;
  les esters de phytostérol ;
- les esters de pentaérythritol ;
- les esters formés à partir :
  - d'au moins un alcool en $C_{16}$-$_{40}$, l'un au moins des alcools étant un alcool de Guerbet et
    d'un dimère diacide formé à partir d'au moins un acide gras insaturé en C18-40,

comme l'ester de dimère d'acides gras de tallol comprenant 36 atomes de carbone et d'un mélange i) d'alcools de Guerbet comprenant 32 atomes de carbone et ii) d'alcool béhénylique ; l'ester de dimère d'acide linoléique et d'un mélange de deux alcools de Guerbet, le 2-tétradécyl-octadécanol (32 atomes de carbone) et le 2-hexadécyl-eicosanol (36 atomes de carbone) ;

les polyesters non réticulés résultant de la polycondensation entre un acide dicarboxylique ou un acide polycarboxylique, linéaire ou ramifié, en $C_4$-$C_{50}$, et un diol ou un polyol en $C_2$-$C_{50}$ ;

les polyesters qui résultent de l'estérification entre un acide polycarboxylique et un ester d'acide carboxylique hydroxylé aliphatique comme le Risocast DA-L et le Risocast DA-H commercialisés par la société japonaise KOKYU ALCOHOL KOGYO, qui sont des esters résultant de la réaction d'estérification de l'huile de ricin hydrogénée avec l'acide dilinoléïque ou l'acide isostéarique ; et

- les esters aliphatiques d'ester résultant de l'estérification entre un ester d'acide carboxylique hydroxylé aliphatique et un acide carboxylique aliphatique, par exemple celui vendu sous la dénomination commerciale Salacos HCIS (V)-L par la société Nishing Oil.

  Un alcool de Guerbet est le produit réactionnel de la réaction de Guerbet bien connue de l'homme du métier. Il s'agit d'une réaction transformant un alcool aliphatique primaire en son alcool dimère -alkylé avec perte d'un équivalent d'eau.

Les acides carboxyliques aliphatiques décrits ci-dessus comprennent généralement de 4 à 30 et de préférence de 8 à 30 atomes de carbone. Ils sont choisis de préférence parmi l'acide héxanoïque, l'acide heptanoïque, l'acide octanoïque, l'acide 2-éthylhexanoïque, l'acide nonanoïque, l'acide décanoïque, l'acide undécanoïque, acide dodécanoïque, l'acide tridécanoïque, l'acide tétradécanoïque, l'acide pentadécanoïque, l'acide hexadécanoïque, l'acide hexyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

Les acides carboxyliques aliphatiques sont de préférence ramifiés.

Les esters d'acide carboxylique aliphatique hydroxylé sont avantageusement issus d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. Les esters d'acide carboxylique aliphatique hydroxylé sont notamment choisis parmi :

a) les esters, partiels ou totaux, d'acides monocarboxyliques aliphatiques monohydroxylés linéaires, saturés ;
b) les esters, partiels ou totaux, d'acides monocarboxyliques aliphatiques monohydroxylés insaturés ;
c) les esters, partiels ou totaux, de polyacides carboxyliques aliphatiques monohydroxylés saturés ;
d) les esters, partiels ou totaux, de polyacides carboxyliques aliphatiques polyhydroxylés saturés ;
e) les esters, partiels ou totaux, de polyols aliphatiques en $C_2$ à $C_{16}$ ayant réagi avec un acide mono- ou un polycarboxylique aliphatique mono-ou polyhydroxylé,
f) et leurs mélanges.

Les esters aliphatiques d'ester sont avantageusement choisis parmi :

- l'ester résultant de la réaction d'estérification de l'huile de ricin hydrogénée avec l'acide isostéarique dans les proportions 1 pour 1 (1/1), qui est appelé monoisostéarate d'huile de ricin hydrogénée,
- l'ester résultant de la réaction d'estérification de l'huile de ricin hydrogénée avec l'acide isostéarique dans les proportions 1 pour 2 (1/2), qui est appelé le diisostéarate d'huile de ricin hydrogénée,
- l'ester résultant de la réaction d'estérification de l'huile de ricin hydrogénée avec l'acide isostéarique dans les proportions 1 pour 3 (1/3), qui est appelé le triisostéarate d'huile de ricin hydrogénée,
- et leurs mélanges.

De préférence, le composé pâteux est choisi parmi les composés d'origine végétale.

Parmi ceux-ci, on peut notamment citer l'huile de jojoba isomérisée telle que l'huile de jojoba partiellement hydrogénée isomérisée trans fabriquée ou commercialisée par la société Desert Whale sous la référence commerciale Iso-Jojoba-50®, la cire d'orange comme, par exemple, celle qui est commercalisée sous la référence Orange Peel Wax par la société Koster Keunen, le beurre de karité, l'huile d'olive partiellement hydrogénée comme, par exemple, le composé commercialisé sous la référence Beurrolive par la société Soliance, le beurre de cacao, l'huile de mangue comme, par exemple, la Lipex 302 de la société Aarhuskarlshamn.

Le ou les composés pâteux sont présents de préférence en une quantité supérieure ou égale à 1% en poids par rapport au poids total de la composition, par exemple de 1 à 15% en poids, mieux en une quantité supérieure ou

égale à 2% en poids, allant par exemple de 2 à 10 % en poids, et encore plus préférentiellement de 3 à 8 % en poids, par rapport au poids total de la composition.

*Tensioactifs non siliconés*

La première composition selon l'invention peut également comprendre, outre le(s) tensioactif(s) siliconé(s), des agents tensioactifs émulsionnants non siliconés présents notamment en une proportion allant de 0,1 à 20 %, et mieux de 0,3 % à 15 % en poids par rapport au poids total de la composition.

Ces agents tensioactifs peuvent être choisis parmi des agents tensioactifs non siliconés non ioniques, anioniques, cationiques, amphotériques ou encore des émulsionnants tensioactifs.

Les tensioactifs non siliconés utilisés préférentiellement dans la composition selon l'invention sont choisis parmi :

a) les tensioactifs non siliconés non ioniques de HLB supérieur ou égal à 8 à 25˚C, utilisés seuls ou en mélange; on peut citer notamment :

- les esters et éthers d'oses tels que le mélange de cétylstéaryl glucoside et d'alcools cétylique et stéarylique comme le Montanov 68 de Seppic;
- les éthers oxyéthylénés et/ou oxypropylénés (pouvant comporter de 1 à 150 groupes oxyéthylénés et/ou oxypropylénés) de glycérol ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool cétéarylique à 30 groupes oxyéthylénés (nom CTFA "Ceteareth-30"), l'éther oxyéthyléné de l'alcool stéarylique à 20 groupes oxyéthylénés (nom CTFA "Steareth-20"), et l'éther oxyéthyléné du mélange d'alcools gras en C12-C15 comportant 7 groupes oxyéthylénés (nom CTFA "C12-15 Pareth-7") commercialisé sous la dénomination NEODOL 25-7® par SHELL CHEMICALS,
- les esters d'acide gras (notamment d'acide en C8-C24, et de préférence en C16-C22) et de polyéthylène glycol (pouvant comprendre de 1 à 150 motifs d'éthylèneglycol) tels que le stéarate de PEG-50 et le monostéarate de PEG-40 commercialisé sous le nom MYRJ 52P® par la société ICI UNIQUEMA,
- les esters d'acide gras (notamment d'acide en C8-C24, et de préférence en C16-C22) et des éthers de glycérol oxyéthylénés et/ou oxypropylénés (pouvant comporter de 1 à 250 groupes oxyéthylénés et/ou oxypropylénés), comme le monostéarate de PEG-200 glycéryle vendu sous la dénomination Simulsol 220 TM® par la société SEPPIC ; le stéarate de glycéryle polyéthoxylé à 30 groupes d'oxyde d'éthylène comme le produit TAGAT S® vendu par la société GOLDSCHMIDT, l'oléate de glycéryle polyéthoxylé à 30 groupes d'oxyde d'éthylène comme le produit TAGAT O® vendu par la société GOLDSCHMIDT, le cocoate de glycéryle polyéthoxylé à 30 groupes d'oxyde d'éthylène comme le produit VARIONIC LI 13® vendu par la société SHEREX, l'isostéarate de glycéryle polyéthoxylé à 30 groupes d'oxyde d'éthylène comme le produit TAGAT L® vendu par la société GOLDSCHMIDT et le laurate de glycéryle polyéthoxylé à 30 groupes d'oxyde d'éthylène comme le produit TAGAT I® de la société GOLDSCHMIDT,
- les esters d'acide gras (notamment d'acide en C8-C24, et de préférence en C16-C22) et des éthers de sorbitol oxyéthylénés et/ou oxypropylénés (pouvant comporter de 1 à 150 groupes oxyéthylénés et/ou oxypropylénés), comme le polysorbate 20 vendu sous la dénomination Tween 20® par la société CRODA, le polysorbate 60 vendu sous la dénomination Tween 60® par la société CRODA,
- les copolymères d'oxyde propylène et d'oxyde d'éthylène, également appelés polycondensats OE/OP,
- et leurs mélanges.

  Les polycondensats OE/OP sont plus particulièrement des copolymères consistant en des blocs polyéthylène glycol et polypropylène glycol, comme par exemple les polycondensats tribloc polyéthylène glycol/polypropylène glycol/polyéthylène glycol. Ces polycondensats tribloc ont par exemple la structure chimique suivante:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

  formule dans laquelle a va de 2 à 120, et b va de 1 à 100.

  Le polycondensat OE/OP a de préférence un poids moléculaire moyen en poids allant de 1000 à 15000, et de mieux allant de 2000 à 13000. Avantageusement, ledit polycondensat OE/OP a une température de trouble, à 10 g/l en eau distillée, supérieure ou égale à 20 ˚C, de préférence supérieure ou égale à 60 ˚C. La température de trouble est mesurée selon la norme ISO 1065. Comme polycondensat OE/OP utilisable selon l'invention, on peut citer les polycondensats tribloc polyéthylène glycol / polypropylène glycol / polyéthylène glycol vendus sous les dénominations SYNPERONIC® comme les SYNPERONIC PE/ L44® et SYNPERONIC PE/F127® par

la société ICI.

b) les tensioactifs non siliconés non ioniques de HLB inférieur à 8 à 25˚C, éventuellement associés à un ou plusieurs agents tensioactif non ioniques de HLB supérieur à 8 à 25 ˚C, tels que cités ci-dessus tels que :

- les esters et éthers d'oses tels que les stéarate de sucrose, cocoate de sucrose, stéarate de sorbitan et leurs mélanges comme l'Arlatone 2121® commercialisé par la société ICI ;
- les éthers oxyéthylénés et/ou oxypropylénés (pouvant comporter de 1 à 150 groupes oxyéthylénés et/ou oxypropylénés) d'alcools gras (notamment d'alcool en C8-C24, et de préférence en C12-C18) tels que l'éther oxyéthyléné de l'alcool stéarylique à 2 groupes oxyéthylénés (nom CTFA "Steareth-2") ;
- les esters d'acides gras (notamment d'acide en C8-C24, et de préférence en C16-C22) et de polyol, notamment de glycérol ou de sorbitol, tels que stéarate de glycéryle, stéarate de glycéryle tel que le produit vendu sous la dénomination TEGIN M® par la société GOLDSCHMIDT, laurate de glycéryle tel que le produit vendu sous la dénomination IMWITOR 312® par la société HULS, stéarate de polyglycéryl-2, tristéarate de sorbitan, ricinoléate de glycéryle ;
- les lécithines, telles que les lécithines de soja (comme Emulmetik 100 J de Cargill, ou Biophilic H de Lucas Meyer) ;

c) les tensioactifs non siliconés anioniques tels que :

- les sels d'acides gras en C16-C30 notamment ceux dérivant des amines, comme le stéarate de triéthanolamine et/ou le stéarate d'amino-2-méthyl-2-propane di-ol-1,3 ;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ou le phosphate de monocétyle monopotassique (Amphisol K de Givaudan) ;
- les sulfosuccinates tels que le "Disodium PEG-5 citrate lauryl sulfosuccinate" et le "Disodium ricinoleamido MEA sulfosuccinate" ;
- les alkyléthersulfates tels que le lauryl éther sulfate de sodium;
- les iséthionates ;
- les acylglutamates tels que le "Disodium hydrogenated tallow glutamate" (AMISOFT HS-21 R® commercialisé par la société AJINOMOTO) et le sodium stearoyl glutamate (AMISOFT HS-11 PF® commercialisé par la société AJINOMOTO) et leurs mélanges ;
- les dérivés de soja comme le potassium soyate ;
- les citrates, comme le Glyceryl stearate citrate (Axol C 62 Pellets de Degussa) ;
- les dérivés de proline, comme le Sodium palmitoyl proline (Sepicalm VG de Seppic), ou le Mélange de Sodium palmitoyl sarcosinate, Magnesium palmitoyl glutamate, Palmitic acid et Palmitoyl proline (Sepifeel One de Seppic) ;
- les lactylates, comme le Sodium stearoyl lactylate (Akoline SL de Karlshamns AB) ;
- les sarcosinates, comme le sodium palmitoyl sarcosinate (Nikkol sarcosinate PN) ou le mélange de Stéaroyl sarcosine et Myristoyl sarcosine 75/25 (Crodasin SM de Croda) ;
- les sulfonates, comme le Sodium C14-17 alkyl sec sulfonate (Hostapur SAS 60 de Clariant) ;
- les glycinates, comme le sodium cocoyl glycinate (Amilite GCS-12 d'Ajinomoto).

Les compositions conformes à l'invention peuvent également contenir un ou plusieurs tensioactifs non siliconés amphotériques comme les N-acyl-aminoacides tels que les N-alkyl-aminoacétates et le cocoamphodiacetate disodique et les oxydes d'amines tels que l'oxyde de stéaramine.

Le tensioactif utilisable peut également être un tensioactif polymérique, notamment un polymère thermogélifiant.

Selon un mode de réalisation avantageux, la première composition selon l'invention comprend comme tensioactif additionnel non siliconé l'association suivante :

d'au moins un éther d'alcool gras en $C_8$-$C_{24}$ et de polyéthylène glycol, ledit éther comprenant de 1 à 19 motifs oxyéthylène et présentant une HLB< 8 à 25˚C,

d'au moins un éther d'alcool gras en $C_8$-$C_{24}$ et de polyéthylène glycol, ledit éther comprenant de 20 à 1000 motifs oxyéthylène et de HLB > 8 à 25˚C, et

d'au moins un ester d'acide gras en C8-C24 et d'éthers de glycérol oxyéthylénés et/ou oxypropylénés pouvant comporter de 1 à 250 groupes oxyéthylénés et/ou oxypropylénés.

*Matière colorante*

La première composition selon l'invention peut également comprendre au moins une matière colorante comme les matières pulvérulentes, les colorants liposolubles, les colorants hydrosolubles.
Les matières colorantes pulvérulentes peuvent être choisies parmi les pigments et les nacres.
Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C Orange 5, le jaune quinoléine, le rocou.
Ces matières colorantes peuvent être présentes en une teneur allant de 0,01 à 30 % en poids par rapport au poids total chaque composition les comprenant.
De préférence la première composition ne comprend pas de « composé apte à conférer à la seconde composition un caractère filant dmax supérieur ou égal à 5 mm», tel que défini plus loin, ou en comprend en une teneur inférieure ou égale à 1% en poids par rapport au poids de la première composition, de préférence inférieure ou égale à 0,5% en poids et mieux inférieure ou égale à 0,2% en poids.

**II) Seconde composition**

La seconde composition selon l'invention est une composition qui, par sa formulation chimique, est difficile à démaquiller.
Par « composition difficile à démaquiller », on entend une composition qui, lorsqu'elle est appliquée seule sur les cils, n'est pas démaquillée à l'aide des démaquillants classiques comprenant une phase aqueuse, car elle peut comprendre notamment des polymères filmogènes insolubles en solution aqueuse.
Selon un premier mode de réalisation avantageux, la seconde composition comprend un milieu aqueux, constituant une phase aqueuse, et au moins un polymère filmogène dispersé dans ladite phase aqueuse sous forme de particules tel que décrit ci-dessus pour la première composition, le polymère étant présent en une teneur au moins égale à 5% en poids en matières sèches.
De préférence, dans le cas où la seconde composition comprend une telle dispersion de polymère, elle présente une phase aqueuse continue.
De préférence, dans le cas où la seconde composition comprend une telle dispersion de polymère, elle ne comprend pas de « composé apte à conférer à la seconde composition un caractère filant dmax supérieur ou égal à 5 mm», tel que défini plus loin, ou en comprend en une teneur inférieure ou égale à 1% en poids par rapport au poids de la première composition, de préférence inférieure ou égale à 0,5% en poids et mieux inférieure ou égale à 0,2% en poids.
De préférence, dans le cas où la seconde composition comprend une telle dispersion de polymère, la première composition, qui est appliquée avant la seconde, ne comprend pas de dispersion de polymère filmogène dans une phase aqueuse sous forme de particules tel que défini ci-dessus, ou en comprend en une teneur inférieure ou égale à 1 % en poids en matières sèches par rapport au poids de la première composition, de préférence inférieure ou égale à 0,5% en poids en matières sèches et mieux inférieure ou égale à 0,2% en poids en matières sèches.
Selon un second mode de réalisation la composition comprend au moins un polymère filmogène liposoluble. Ce polymère peut être filmogène à température ambiante ou à une température supérieure ne dépassant pas 100°C.
A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).
Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.
Comme exemples de ces copolymères, on peut citer les copolymères : acétate de vinyle/stéarate d'allyle,

l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/ octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/ laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C2-C20, comme le polybutène, les alkylcelluloses avec un radical alkyle linéaire ou ramifié, saturé ou non en C1 à C8 comme l'éthylcellulose et la propylcellulose, les copolymères de la vinylpyrolidone (VP) et notamment les copolymères de la vinylpyrrolidone et d'alcène en C2 à C40 et mieux en C3 à C20. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

On peut également citer les résines de silicone, généralement solubles ou gonflables dans les huiles de silicone, qui sont des polymères de polyorganosiloxanes réticulés. La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

A titre d'exemples de résines polymethylsilsesquioxanes commercialement disponibles, on peut citer celles qui sont commercialisés :

par la société Wacker sous la référence Resin MK tels que la Belsil PMS MK :
par la société SHIN-ETSU sous les références KR-220L.

Comme résines siloxysilicates, on peut citer les résines trimethylsiloxysilicate (TMS) telles que celle commercialisées sous la référence SR1000 par la société General Electric ou sous la référence TMS 803 par la société Wacker. On peut encore citer les résines timéthylsiloxysilicate commercialisées dans un solvant tel que la cyclomethicone, vendues sous la dénomination "KF-7312J" par la société Shin-Etsu, "DC 749", "DC 593" par la société Dow Corning.

On peut aussi citer des copolymères de résines de silicone telles que celles citées ci-dessus avec des polydiméthylsiloxanes, comme les copolymères adhésifs sensibles à la pression commercialisés par la société Dow Corning sous la référence BIO-PSA et décrits dans le document US 5 162 410 ou encore les copolymères siliconés issus de la réaction d'un résine de silicone, telle que celles décrite plus haut, et d'un diorganosiloxane tels que décrits dans le document WO 2004/073626.

De préférence, dans le cas où la seconde composition comprend un polymère filmogène liposoluble, elle présente une phase huileuse continue.

De préférence, dans le cas où la seconde composition comprend un tel polymère filmogène, elle ne comprend pas de « composé apte à conférer à la seconde composition un caractère filant dmax supérieur ou égal à 5 mm», tel que défini plus loin, ou en comprend en une teneur inférieure ou égale à 1% en poids par rapport au poids de la première composition, de préférence inférieure ou égale à 0,5% en poids et mieux inférieure ou égale à 0,2% en poids.

La seconde composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

Selon un troisième mode de réalisation avantageux, la seconde composition selon l'invention comprend au moins un composé apte à conférer à la seconde composition un caractère filant dmax supérieur ou égal à 5 mm.

De préférence, dans ce cas, la seconde composition selon l'invention est anhydre. Par « anhydre », on entend une composition comprenant moins de 10% d'eau en poids par rapport au poids total de la composition, de préférence

moins de 5%. De préférence la seconde composition est exempte d'eau.

1) Mesure du caractère filant

Selon un premier mode de réalisation avantageux, la seconde composition selon l'invention comprend au moins un composé apte à conférer à la seconde composition un caractère filant dmax supérieur ou égal à 5 mm.

Le caractère filant de la seconde composition est déterminé à l'aide du texturomètre vendu sous la dénomination TA X- T2i par la société RHEO, équipé d'un mobile controlé en température, ce mobile étant une cartouche chauffante en inox de référence Firerod DIV-STL (Société Watlow, France), de diamètre 3.17 mm et de longueur 60 mm, d'une puissance maximale de 40W sous une tension de 24V, avec un thermocouple type KlocC. La cartouche chauffante est alimentée par une source de courant continue 5V / 0.5A LKS 005-5V de Elka-Electronique. Sa température est régulée par un contrôleur PID TC48 de Faucigny instrument (France). Un appendice de fixation a été créé pour fixer le mobile contrôlé en température sur le bras de mesure du texturomètre.

La mesure est faite sur des fils de composition obtenu en imposant un déplacement vertical du mobile jusqu'au contact avec un échantillon de la composition puis, après un temps d'attente au contact, en imposant un déplacement vertical du mobile vers le haut. La composition ayant un caractère filant à chaud, un fil se forme entre le mobile en phase de retrait et l'échantillon de la composition, lequel fil devenant plus consistant sous l'effet du refroidissement à l'air ambiant. La mesure de dmax consiste en une mesure de la longueur des fils ainsi formés après détachement de la surface du mobile.

Le protocole est le suivant :

a) on prépare un échantillon de la composition en remplissant à son maximum une coupelle en inox de 2 mm d'épaisseur et 20 mm de diamètre, l'excès de composition étant arasé en surface,
b) on contrôle la température du mobile à 40˚C,
c) le mobile descend à une vitesse de 10 mm/s jusqu'au contact de la surface de la composition,
d) le mobile est maintenu fixe pendant 10 s puis est relevé à une vitesse de 10 mm/s.

Pendant la phase de retrait du mobile, un fil est formé entre la composition et le mobile. Au fur et à mesure que le mobile est éloigné de la surface de la composition, le fil formé refroidit et devient plus consistant. A partir d'une certaine élongation, le fil se détache du mobile.

Le caractère filant ou dmax (exprimé en mm) correspond à la longueur du fil obtenu après rupture, mesurée avec une règle graduée.

La mesure du caractère filant est répétée trois fois pour la même composition, en différents endroits de la coupelle, et une moyenne de « filant » dmax est calculée pour chaque composition.

Les étapes b) à d) sont répétées pour la même composition à une température de mobile fixée à l'étape b) respectivement de 50˚C, de 60˚C, de 70˚C, de 80˚C, de 90˚C, de 100˚C, de 110˚C, de 120˚C, de 130˚C et de 140˚C.

Parmi les valeurs de filant pouvant être obtenues aux différentes températures, on retient comme valeur de caractère filant dmax la valeur la plus élevée.

La seconde composition utilisée dans la procédé selon l'invention présente un caractère filant dmax supérieur ou égal à 5 mm, pouvant aller jusqu'à 100 mm, de préférence supérieur ou égal à 7 mm, mieux supérieur ou égal à 10 mm et mieux supérieur ou égal à 15 mm.

De préférence, la seconde composition est apte à former un fil tel que, si après formation du fil et mesure du dmax selon le protocole indiqué ci-dessus, on place la coupelle comprenant la composition à la verticale (de manière à ce que le fil soit en position horizontale, c'est-à-dire soumis à la gravité) au moins 30 secondes, le fil conserve une longueur minimale de 5 mm (mesurable manuellement à la règle graduée).

La seconde composition présentant un tel caractère filant selon l'invention permet l'obtention, lors de l'application sur les fibres kératiniques, d'un fil de composition dans le prolongement du cil. Ce fil conserve sa forme, reste rigide et ne se rétracte pas, ce qui permet l'obtention d'un effet d'allongement du cil.

La seconde composition mise en oeuvre dans le procédé selon l'invention est chauffée à une température supérieure ou égale à 40˚C, de préférence supérieure ou égale à 45˚C, mieux supérieure ou égale à 50˚C, et encore mieux, supérieure ou égale à 60 ˚C.

La température peut aller jusqu'à 150˚C, de préférence jusqu'à 120˚C, mieux jusqu'à 100˚C, encore mieux jusqu'à 95˚C.

Selon un mode particulier, la seconde composition est portée à une température allant jusqu'à 150˚C, de préférence jusqu'à 120˚C, mieux jusqu'à 100˚C, encore mieux jusqu'à 95˚C.

De préférence, la seconde composition est portée à la température à laquelle elle présente le caractère filant d max, mesuré comme indiqué précédemment (c'est-à-dire à la température à laquelle le caractère filant est le plus élevé).

2) Composé apte à conférer à la seconde composition un caractère filant dmax supérieur ou égal à 5 mm

La composition comprend avantageusement au moins un composé conférant à ladite composition un caractère filant dmax supérieur ou égal à 5 mm ou un mélange de composés tel que ledit mélange confère à ladite composition un caractère filant dmax supérieur ou égal à 5 mm, lorsque celle-ci est chauffée à une température supérieure ou égale à 40˚C.

Ce composé peut être hydrocarboné ou siliconé et présente avantageusement un comportement thermoplastique.

Ce composé est de préférence solide à température ambiante. Avantageusement, il présente un caractère filant dmax supérieur ou égal à 5 mm lorsqu'il est porté à une température supérieure ou égale à'40˚C, c'est-à-dire qu'il est apte à produire des fils tels que décrits plus haut, à une température supérieure ou égale à 40˚C, par exemple allant de 40 à 150˚C, de préférence supérieure ou égale à 45˚C, par exemple allant de 45 à 120˚C, mieux supérieure ou égale à 50˚C, pax exemple allant de 50 à 100˚C et encore mieux, supérieure ou égale à 60 ˚C

Ce composé est de préférence un polymère et peut être avantageusement choisi parmi :

A/ Les polymères et copolymères comprenant au moins un monomère alcène, en particulier les copolymères à base d'éthylène

De tels composés peuvent être choisis parmi:

- les copolymères d'alcène et d'acétate de vinyle, en particulier les copolymères d'éthylène et d'acétate de vinyle. On utilise en particulier les copolymères d'éthylène et d'acétate de vinyle comprenant de préférence entre 5 et 50% en poids d'acétate de vinyle par rapport au poids total du polymère, de préférence entre 10 et 45% en poids, de préférence entre 20 et 40% en poids.
  Comme exemples de copolymères ethylène/acétate de vinyle, on peut citer ceux qui sont commercialisés sous la dénomination ELVAX par la société Du Pont de Nemours et en particulier les composés Elvax 40W, Elvax 140W, Elvax 200W, Elvax 205W, Elvax 210W et Elvax 310.
  On peut également citer les produits commercialisés sous la dénomination EVATANE par la société Arkema tels que l'Evatane 28-800. On peut encore citer le MELTHENE - H Grade H-6410M proposé par la société Tosoh Polymer.
- les copolymères d'éthylène et d'octène tels que par exemple les produits commercialisés sous la référence « AFFINITY» par la société Dow Plastics comme par exemple l'AFFINITY GA 1900 GA 1950 ;
- et leurs mélanges.

Ces polymères et copolymères peuvent être utilisés seuls ou en mélange avec au moins un composé choisi parmi les résines dites tackifiantes telles que décrites dans le Handbook of Pressure Sensitive Adhesive, edited by Donatas Satas, 3rd ed., 1989, p. 609-619, les cires, et leurs associations. Les résines tackifiantes peuvent être notamment choisies parmi la colophane (correspondant au terme anglo-saxon « rosin »), les dérivés de colophane, les résines hydrocarbonées et leurs mélanges.

On peut citer en particulier les résines hydrocarbonées indéniques telles que les résines issues de la polymérisation en proportion majoritaire de monomère indène et en proportion minoritaire de monomères choisis parmi le styrène, le méthylindène, le méthylstyrène et leurs mélanges. Ces résines peuvent éventuellement être hydrogénées. Elles peuvent présenter un poids moléculaire allant de 290 à 1150.

Comme exemples de résines indéniques, on peut citer en particulier les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.

Comme mélange à base de copolymère éthylène/acétate de vinyle, on peut citer par exemple les produits commercialisés sous la dénomination Coolbind par la société National Starch.

Ces polymères peuvent se présenter sous forme pure ou être véhiculés dans une phase aqueuse ou une phase solvant organique.

B/ Les homopolymères polyacétates de vinyle, présentant de préférence un poids moléculaire inférieur à 20000 comme par exemple le RAVIFLEX BL1S de la société Vinavil.

C/ Les résines siliconées

Ces résines sont des polymères d'organosiloxanes réticulés.

La nomenclature des résines de silicone est connue sous le « nom » de "MDTQ", la résine étant décrite en fonction des différentes unités monomériques siloxane qu'elle comprend, chacune des « lettres » "MDTQ" caractérisant du type d'unité.
La lettre M représente l'unité monofonctionelle de formule $(CH_3)_3S'O_{1/2}$, l'atome de silicium étant relié à un seul atome d'oxygène dans le polymère comprenant cette unité.
La lettre D signifie une unité difonctionnelle $(CH_3)_2SiO_{2/2}$ dans laquelle l'atome de silicium est relié à deux atomes d'oxygène
La lettre T représente une unité trifonctionnelle de formule $(CH_3)SiO_{3/2}$.
Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.
Enfin, la lettre Q signifie une unité tetrafonctionnelle $SiO_{4/2}$ dans laquelle l'atome de silicium est lié à quatre atomes d'oxygène eux mêmes liés au reste du polymère.

On peut cite en particulier les résines T, notamment les résines de silicone T fonctionnalisées telles que les polyphenylsiloxanes, en particulier fonctionnalisées par des groupes silanols (Si-OH), comme celle commercialisées sous la référence Dow Corning (R) Z-1806.

D/ les polymères éthyléniques séquencés filmogènes

Ces polymères comprennent de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.
Avantageusement, les première et deuxième séquences et du polymère séquencé sont incompatibles l'une avec l'autre.
De tels polymères sont décrits par exemple dans les documents EP 1411069 ou WO04/028488.
Par polymère "séquencé", on entend un polymère comprenant au moins 2 séquences distinctes, par exemple au moins 3 séquences distinctes.
Les première et deuxième séquences du polymère se distinguent l'une de l'autre par leur degré de déformabilité. Ainsi, la première séquence peut être rigide et la deuxième séquence peut être souple.
Les températures de transition vitreuse des séquences souple et rigide peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_{i} (\acute{\omega}_i / Tg_i) ,$$

$\acute{\omega}_i$, étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.
La séquence rigide peut avoir une Tg supérieure à 20 ˚C,
La séquence souple peut avoir une Tg inférieure ou égale à 20 ˚C.
Selon un mode de réalisation, le copolymère comprend une première séquence rigide et une deuxième séquence souple.
De préférence, la proportion de la séquence rigide va de 20 à 90 % en poids du copolymère, mieux de 30 à 90 % et encore mieux de 50 à 90 %.
De préférence, la proportion de la séquence souple va de 5 à 75 % en poids du copolymère, de préférence de 10 à 50 % et mieux de 15 à 45 %.

<u>Séquence rigide</u>

Dans le cadre de la présente invention, la ou les séquences rigides sont plus particulièrement formées à partir de monomères suivants :

- les méthacrylates de formule $CH_2 = C(CH_3)\text{-}COOR_1$ dans laquelle $R_1$ représente un groupe alkyle non substitué, linéaire ou ramifié, en $C_1$ à $C_4$, tel qu'un groupe méthyle, éthyle, propyle ou isobutyle ou $R_1$ représente un groupe cycloalkyle $C_4$ à $C_{12}$ tel qu'un groupe isobornyle,

- les acrylates de formule $CH_2$ = CH-$COOR_2$ dans laquelle $R_2$ représente un groupe tertio butyle ou un groupe cycloalkyle en $C_4$ à $C_{12}$ tel qu'un groupe isobornyle,
- les (méth)acrylamides de formule:

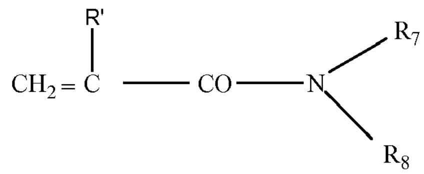

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, iso ctyle, ou isononyle ; ou $R_7$ représente H et $R_8$ représente un groupement 1,1-diméthyl-3-oxobutyl,
et R' désigne H ou méthyle.
Comme exemple de monomères de ce type, on peut citer le N-butylacrylamide, le N-t-butylacrylamide, le N-isopropylacrylamide, le N,N-diméthylacrylamide et le N,N-dibutylacrylamide ,

- et leurs mélanges.

Des monomères de la séquence rigide particulièrement préférés sont le méthacrylate d'isobornyle, l'acrylate d'isobornyle et leurs mélanges.

Séquence souple

Dans le cadre de la présente invention, la ou les séquences souples sont plus particulièrement formées à partir de monomères suivants :

- les acrylates de formule $CH_2$ = $CHCOOR_3$, avec $R_3$ représentant un groupe alkyle non substitué en $C_1$ à $C_{12}$, linéaire ou ramifié, tel qu'un groupe isobutyle (à l'exception d'un groupe tertiobutyle), dans lequel se trouve(nt) éventuellement intercalé(s) un ou plusieurs hétéroatomes choisis parmi O, N, S,
- les méthacrylates de formule $CH_2$ = C($CH_3$)-$COOR_4$, avec $R_4$ représentant un groupe alkyle non substitué en $C_6$ à $C_{12}$ linéaire ou ramifié,

dans lequel se trouve(nt) éventuellement intercalé(s) un ou plusieurs hétéroatomes choisis parmi O, N et S;

- les esters de vinyle de formule $R_5$-CO-O-CH = $CH_2$ où $R_5$ représente un groupe alkyle en $C_4$ à $C_{12}$ linéaire ou ramifié ;
- les éthers de vinyle et d'alkyle en $C_4$ à $C_{12}$,
- et leurs mélanges.

Des monomères de la séquence souple particulièrement préférés sont l'acrylate d'isobutyle.
Chacune des séquences peut contenir en proportion minoritaire au moins un monomère constitutif de l'autre séquence.
Ainsi la première séquence peut contenir au moins un monomère constitutif de la deuxième séquence et inversement.
Chacune des première et/ou deuxième séquence, peu(ven)t comprendre, outre les monomères indiqués ci-dessus, un ou plusieurs autres monomères appelés monomères additionnels, différents des monomères principaux cités précédemment.
Ce monomère additionnel est par exemple choisi parmi :

a)les monomères hydrophiles tels que :

- les monomères à insaturation(s) éthylén'que(s), autre que l'acide acrylique, comprenant au moins une fonction acide carboxylique ou sulfonique comme par exemple l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,

- les méthacrylates de formule $CH_2 = C(CH_3)\text{-}COOR_6$ dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,

- les méthacrylates de formule $CH_2 = C(CH_3)\text{-}COOR_9$, $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule $CH_2 = CHCOOR_{10}$, $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle ($C_1$-$C_{12}$)-O-POE (polyoxyéthylène) avec répétition du motif oxyéthylène de 5 à 30 fois, par exemple méthoxy-POE, ou $R_{10}$ représente un groupement polyoxyéthyléné comprenant de 5 à 30 motifs oxyde d'éthylène

b) les monomères à insaturation(s) éthylénique(s) comprenant un ou plusieurs atomes de silicium tels que le méthacryloxypropyl triméthoxy silane, le méthacryloxypropyl tris (triméthylsiloxy) silane,

- et leurs mélanges.

Ce ou ces monomères additionnels représente(nt) généralement une quantité inférieure ou égale à 30 % en poids, par exemple de 1 à 30 % en poids, de préférence de 5 à 20 % en poids et, de préférence encore, de 7 à 15 % en poids du poids total des première et/ou deuxième séquences.

Selon un mode de mise en oeuvre, le copolymère peut comprendre au moins une première séquence et au moins une deuxième séquence reliées entre elles par un segment intermédiaire comprenant au moins un monomère constitutif de la première séquence et au moins un monomère constitutif de la deuxième séquence.
De préférence, la séquence intermédiaire est issue essentiellement de monomères constitutifs de la première séquence et de la deuxième séquence.
Avantageusement, le segment intermédiaire comprenant au moins un monomère constitutif de la première séquence et au moins un monomère constitutif de la deuxième séquence du copolymère est un polymère statistique.
Avantageusement, le copolymère est issu essentiellement de monomères choisis parmi les méthacrylates d'alkyle, les acrylates d'alkyle, et leurs mélanges.
Par « essentiellement », on entend, dans ce qui précède et dans ce qui suit, comprenant au moins 85 %, de préférence au moins 90 %, mieux au moins 95 % et encore mieux 100 %.
En ce qui concerne les esters acrylates et méthacrylates, ils peuvent dériver de l'estérification d'alcools linéaire ou ramifié, cyclique ou aromatiques en $C_1$ à $C_{12}$, en particulier en $C_4$ à $C_{10}$.
A titre illustratif et non limitatif de ces alcools, on peut notamment citer l'isoborneol.
Selon un mode de réalisation, ledit copolymère comprend au moins des monomères d'acrylates et de méthacrylates dérivant de l'estérification d'un même alcool et en particulier de l'isobornéol.

De préférence, le polymère séquencé linéaire filmogène comprend au moins des monomères acrylate d'isobornyle au moins des monomères méthacrylate d'isobornyle et au moins des monomères acrylate d'isobutyle.
Selon une variante de réalisation, le polymère séquencé peut comprendre au moins :

- une séquence rigide, qui est un copolymère de méthacrylate d'isobornyle/acrylate d'isobornyle, et
- une séquence souple, qui est un copolymère d'acrylate d'isobutyle.

Plus précisément, le copolymère peut comprendre 50 à 80 % en poids de méthacrylate/acrylate d'isobornyle et de 10 à 20 % en poids d'acrylate d'isobutyle.

La masse moyenne en poids (Mw) du copolymère va de préférence de 80 000 à 300 000, voire de 100 000 à 150 000.
La masse moyenne en nombre (Mn) du copolymère va de préférence
20 000 à 90 000, elle va par exemple de 25 000 à 45 000.

E/ Les copolymères de diènes et de styrène, notamment les copolymères de butadiène et de styrène.

On peut citer notamment les copolymères styrène/butadiène commercialisés sous la référence PLIOLITE S5E

par la société Eliokem.

F/ les polyesters comprenant au moins un monomère portant au moins un groupement - $SO_3M$ (M représentant un atome d'hydrogène, un ion ammonium $NH_4^+$ ou un ion métallique), aussi appelés sulfopolyesters.

Ces polyesters possèdent avantageusement une température de transition vitreuse (Tg) supérieure à 38°C, Ils peuvent présenter une masse moléculaire moyenne en poids avantageusement inférieur à 200 000, par exemple allant de 10 000 à 50 000.

Ces polyesters peuvent être obtenus, de façon connue, par polycondensation d'au moins un acide dicarboxylique avec au moins un polyol, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence n diol choisi parmi : l'éthylène glycol, le diéthylène glycol, le triéthylène glycol, le 1,3-propanediol, le cyclohexane diméthanol, le 4-butanediol.
Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools.
Comme diamine on peut utiliser l'éthylènediamine, l'hexaméthylènediamine, la méta- ou para-phénylènediamine. Comme aminoalcool, on peut utiliser la monoéthanolamine.

Le polyester comprend au moins un monomère portant au moins un groupement - $SO_3M$, avec M représentant un atome d'hydrogène, un ion ammonium $NH_4^+$ ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement -$SO_3M$.

Le noyau aromatique du monomère aromatique bifonctionnel portant en outre un groupement -$SO_3M$ tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -$SO_3M$: l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.
De tels polymères sont vendus par exemple sous le nom de marque Eastman AQ ® par la société NOVEON comme par exemple L'EASTMAN AQ 38S.

G/ Les cires

Les cires peuvent être choisies parmi les cires décrites précédemment.

De préférence, la cire utilisée dans la seconde composition est une cire de paraffine.

H/ les fibres

Par « fibre », il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, en particulier de 5 à 500, et plus particulièrement de 5 à 150.
Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.
En particulier, les fibres ont une longueur allant de 1 $\mu$m à 10 mm, en particulier de 0,1 mm à 5 mm et plus particulièrement

de 0,3 mm à 3,5 mm. Leur section peut être comprise dans un cercle de diamètre allant de 2 nm à 500 µm, en particulier allant de 100 nm à 100 µm et plus particulièrement de 1 µm à 50 µm. Le poids ou titre des fibres est souvent donné en denier ou décitex et représente le poids en gramme pour 9 km de fil. Les fibres selon l'invention peuvent en particulier avoir un titre choisi dans la gamme allant de 0,15 à 30 deniers et notamment de 0,18 à 18 deniers.

Les fibres utilisables dans la composition de l'invention peuvent être choisies parmi les fibres rigides ou non rigides, elles peuvent être d'origine synthétique ou naturelle, minérales ou organiques.

Par ailleurs, les fibres peuvent être traitées ou non en surface, enrobées ou non, colorées ou non colorées.

A titre de fibres utilisables dans la composition selon l'invention, on peut citer les fibres non rigides telles que les fibres de polyamide (Nylon®) ou les fibres rigides telles que les fibres de polyimide-amide comme celles vendues sous les dénomination KERMEL®, KERMEL TECH® par la société RHODIA ou de poly-(p-phénylène-téréphtalamide) (ou d'aramide) notamment vendues sous la dénomination Kevlar ® par la société DUPONT DE NEMOURS.

Selon un mode préféré, la seconde composition comprend au moins un composé ou mélange de composés choisis parmi les polymères et copolymères comprenant au moins un monomère alcène, en particulier les copolymères à base d'éthylène.

En particulier, la seconde composition comprend au moins un composé ou mélange de composés choisis parmi les copolymères d'alcène et d'acétate de vinyle, en particulier les copolymères d'éthylène et d'acétate de vinyle.

Selon un autre mode particulier, la seconde composition comprend au moins un composé ou mélange de composés choisis parmi les copolymères d'éthylène et d'octène.

Selon un mode de réalisation avantageux, la seconde composition comprend au moins un copolymère éthylène/acétate de vinyle.

Selon un mode de réalisation avantageux, la composition comprend comme composés conférant à la composition un caractère filant dmax supérieur ou égal à 5 mm, un mélange de cires, en particulier de cire de paraffine et de copolymère éthylène/acétate de vinyle.

Selon un mode particulier, le mélange de cire et de copolymère d'éthylène et d'acétate de vinyle comprend de 50 à 65% en poids de copolymère éthylène/acétate de vinyle par rapport au poids total du mélange.

Selon un mode particulier, le mélange de cire et de copolymère d'éthylène et d'acétate de vinyle comprend de 35 to 50% en poids de cire de paraffine par rapport au poids total du mélange.

Ledit mélange peut comprendre en particulier de 50 à 65% en poids de copolymère éthylène/acétate de vinyle par rapport au poids total du mélange et de 35 to 50% en poids de cire de paraffine par rapport au poids total du mélange.

De préférence, le copolymère éthylène/acétate de vinyle comprend plus de 25% en poids par rapport au poids total du polymère, d'acétate de vinyle, par exemple environ 28% en poids.

De préférence, le copolymère éthylène/acétate de vinyle présente une masse moléculaire moyenne en poids (Mw) allant de 50000 à 80000, mieux de 60 000 à 70 000, et encore mieux de 63000 à 73000.

Le composé ou le mélange de composés conférant à la composition un caractère filant dmax supérieur ou égal à 5 mm peut être présent dans la composition en une teneur en matières sèches d'au moins 5% en poids par rapport au poids total de la composition, par exemple allant de 5 à 100% en poids par rapport au poids total de la composition, de préférence allant de 10 à 100% en poids et mieux de 15 à 100% en poids.

3) Autres composés

La seconde composition selon l'invention peut comprendre un gélifiant hydrosoluble. Les gélifiants hydrosolubles utilisables dans les secondes compositions selon l'invention peuvent être choisis parmi ceux listés précédemment pour la première composition.

Le polymère gélifiant hydrosoluble peut être présent dans la seconde composition le comprenant en une teneur en matières sèches allant de 0,01 % à 60 % en poids, de préférence de 0,5 % à 40 % en poids, mieux de 1 % à 30 % en poids, voire de 5 à 20 % en poids par rapport au poids total de la seconde composition.

**Huiles**

La première et/ou la seconde composition selon l'invention peut en outre comprendre une ou plusieurs huiles ou corps gras non aqueux liquides à température ambiante (25°C) et pression atmosphérique (760 mm de Hg).

L'huile peut être choisie parmi les huiles volatiles et/ou les huiles non volatiles, et leurs mélanges.

La ou les huiles peuvent être présentes en une teneur allant de 0,1 % à 95 % en poids, de préférence de 0,5 % à 60 % en poids par rapport au poids total de la composition les comprenant.

Par "huile volatile", on entend au sens de l'invention une huile susceptible de s'évaporer au contact des fibres kératiniques en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique,

allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg). Par "huile non volatile", on entend une huile restant sur la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'isohexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes (8 $10^{-6}$ $m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale
(I):

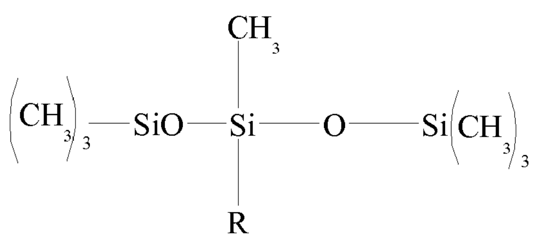

où R représente un groupe alkyle comprenant de 2 à 4 atomes de carbone et dont un ou plusieurs atomes d'hydrogène peuvent être substitués par un atome de fluor ou de chlore. Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.

La première et/ou la seconde composition peut également comprendre au moins une huile non volatile, et notamment choisie parmi les huiles hydrocarbonées et/ou siliconées et/ou fluorées non volatiles.

Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;

- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R1 + R2 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans les compositions selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles fluorées utilisables dans l'invention sont notamment des huiles fluorosiliconées, des polyéthers fluorés, des silicones fluorées telles que décrit dans le document EP-A-847752.

La première et/ou la seconde composition peut également comprendre des ingrédients couramment utilisés en cosmétique tels que les charges, les fibres et leurs mélanges.

La seconde composition peut également comprendre des matières colorantes telles que décrites ci-dessus.

Charges

La première et/ou seconde composition selon l'invention peut comprendre au moins une charge.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® commercialisé sous la dénomination Orgasol® par la société Atochem, de poly-β-alanine et de polyéthylène, les poudres de polymères de tétrafluoroéthylène comme le Téflon®, la lauroyl-lysine, l'amidon, le nitrure de bore, les micro sphères creuses polymériques expansées telles que celles de chlorure de polyvinylidène/acrylonitrile comme celles commercialisées sous la dénomination d'Expancel® par la société Nobel Industrie, les poudres acryliques telles que celles commercialisées sous la dénomination Polytrap® par la société Dow Corning, les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (Silica Beads® de MAPRECOS), les microcapsules de verre ou de céramique, les savons métalliques dérivés d'acides organiques carboxyliques ayant de 8 à 22 atomes de carbone, et en particulier de 12 à 18 atomes de carbone, par exemple le stéarate de zinc, de magnésium ou de lithium, le laurate de zinc, le myristate de magnésium.

On peut également utiliser un composé susceptibles de gonfler à la chaleur et notamment des particules thermoexpansibles telles que les microsphères non expansées de copolymère de chlorure de vinylidène/d'acrylonitrile/méthacrylate de méthyle ou de copolymère d'homopolymère d'acrylonitrile comme par exemple celles commercialisées respectivement sous les références Expancel® 820 DU 40 et Expancel®007WU par la Société AKZO NOBEL.

Les charges peuvent représenter de 0,1 à 25 %, en particulier de 1 à 20 % en poids par rapport au poids total de la composition.

Fibres

La première et/ou la seconde composition selon l'invention peut comprendre, outre le composé ou le mélange de composés qui confère à ladite composition un caractère filant dmax supérieur ou égal à 5 mm, des fibres dites additionnelles. Ces fibres additionnelles seules ne contribuent pas au caractère filant de la composition.

Les fibres additionnelles peuvent être présentes dans la composition selon l'invention en une teneur allant de 0,01 % à 10 % en poids, par rapport au poids total de la composition les comprenant, en particulier de 0,1 % à 5 % en poids, et plus particulièrement de 0,3 % à 3 % en poids.

Actifs cosmétiques

Comme actifs cosmétiques pouvant être utilisés dans les compositions selon l'invention, on peut citer notamment des antioxydants, des conservateurs, des parfums, des neutralisants, des émollients, des hydratants, des vitamines et des filtres en particulier solaires.

Bien entendu, l'homme du métier veillera à choisir les éventuels additifs complémentaires et/ou leur quantité de telle manière que les propriétés avantageuses des compositions selon l'invention ne soient pas, ou substantiellement pas, altérées par l'adjonction envisagée.

Les première et seconde compositions peuvent se présenter sous forme solide, semi-solide ou liquide.

Les première et seconde compositions peuvent se présenter notamment sous forme de suspension, de dispersion, de solution, de gel, d'émulsion, notamment émulsion huile-dans-eau (H/E), cire-dans-eau ou multiple (E/H/E ou polyol/H/E), sous forme de crème, de pâte, de mousse, de dispersion de vésicules notamment de lipides ioniques ou non, de lotion biphase ou multiphase, de spray, de poudre, de pâte, notamment de pâte souple. Chaque composition est de préférence une composition non rinçée.

La seconde composition peut aussi se présenter sous forme d'émulsion eau-dans-huile (E/H), ou multiple (H/E/H).

Les première et seconde compositions selon l'invention peuvent être fabriquées par les procédés connus, généralement utilisés dans le domaine cosmétique.

La première composition peut être appliquée sur les cils à l'aide d'un applicateur de mascara conventionnel, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits, par exemple, dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture. Dans le cadre du procédé selon l'invention, la première composition est notamment appliquée sur les fibres kératiniques à l'aide d'une brosse à mascara.

La première composition se présenter en particulier sous d'émulsion cire-dans-eau, de dispersion aqueuse ou de gel aqueux.

Dans le procédé de l'invention, la seconde composition, lorsqu'elle comprend au moins un composé apte à conférer à ladite composition un caractère filant dmax supérieur ou égal à 5 mm, est généralement chauffée à une température supérieure ou égale à 40 ˚C, notamment supérieure ou égale à 45 ˚C, en particulier supérieure ou égale à 50 ˚C.

Evidemment, la température de chauffage dépend en particulier de la température susceptible d'être supportée par le support traité.

Selon un mode de réalisation, la seconde composition est sous forme solide.

Selon un mode de réalisation, la première composition se présente sous d'émulsion cire-dans-eau, de dispersion aqueuse ou de gel aqueux, et la seconde composition est sous forme solide.

Selon un premier mode de réalisation du procédé selon l'invention, la seconde composition, lorsqu'elle comprend au moins un composé apte à conférer à ladite composition un caractère filant dmax supérieur ou égal à 5 mm, est solide et est chauffée antérieurement à son application, le moyen de chauffage utilisé pouvant être l'applicateur luimême. Ainsi, dans le cas d'un mascara, la seconde composition peut être appliquée à l'aide d'un applicateur chauffant tel qu'une brosse chauffante.

Selon un second mode de réalisation du procédé selon l'invention, la seconde composition, lorsqu'elle comprend au moins un composé apte à conférer à ladite composition un caractère filant dmax supérieur ou égal à 5 mm, est chauffée lors de son application. Dans un tel cas, le moyen de chauffage utilisé est généralement l'applicateur luimême. Ainsi, dans le cas d'un mascara, la seconde composition peut être appliquée à l'aide d'une brosse chauffante.

Selon un troisième mode de réalisation, la seconde composition, lorsqu'elle comprend au moins un composé apte à conférer à ladite composition un caractère filant dmax supérieur ou égal à 5 mm, est chauffée postérieurement à son application. Selon une première variante, la seconde composition peut être chauffée à l'aide de moyens non spécifiquement destinés à un chauffage, comme par exemple un corps occasionnellement chaud. Selon une seconde variante de ce mode de réalisation, la composition peut être chauffée à l'aide d'un moyen spécifiquement dédié au chauffage. Il peut en particulier s'agir d'un moyen propulsant de l'air chaud tel qu'un sèche-cheveux ou un dispositif de chauffage par exemple tel que décrit ci-après.

Selon un mode de réalisation, la seconde composition selon l'invention, lorsqu'elle comprend au moins un composé apte à conférer à ladite composition un caractère filant dmax supérieur ou égal à 5 mm, est sous forme de

particules, de poudre ou de masse pulvérulente. Cette seconde composition peut être appliquée sur les fibres kératiniques à l'aide d'un dispositif d'application comportant un support chauffant, la composition étant contenue dans un embout applicateur ayant une forme adaptée à son montage par emmanchement sur le support chauffant ou la composition étant contenue dans un récipient dans lequel le support chauffant peut être immergé pour se charger en composition. Selon un mode de réalisation, la seconde composition se présentant sous forme de poudre est placée sur la partie chauffante d'un applicateur chauffant, tel qu'une brosse ou un peigne chauffant, jusqu'à ce qu'elle ramollisse, puis elle est appliquée sur les fibres kératiniques.

La seconde composition selon l'invention peut être conditionnée dans un ensemble de conditionnement et d'application comprenant :

i) un réservoir comprenant ladite composition,
ii) un dispositif pour l'application de la composition, et
iii) des moyens de chauffage.

Selon un mode de réalisation, les moyens de chauffage sont formés par un dispositif distinct du dispositif ou organe d'application, l'ensemble étant configuré sous forme d'un dispositif de conditionnement et d'application comprenant en outre un récipient contenant une seconde composition conforme à l'invention. Un tel dispositif peut être conditionné à l'intérieur d'un conditionnement du type blister pack. Les moyens de chauffage peuvent être du type de ceux décrits dans les brevets US 6 009 884 ou US 5 853 010. D'autres dispositifs configurés sous forme d'une pince chauffante (dans le cas des cils) peuvent également être utilisés. De tels dispositifs sont décrits notamment dans le brevet US 6 220 252.

Selon un mode de réalisation le dispositif ou organe d'application comprend des moyens de chauffage de la seconde composition, en particulier les moyens de chauffage associés au dispositif d'application sont agencés de manière à ne pas chauffer de manière sensible au moins une partie de la tige.

Le kit 1 décrit à la figure 1 comprend un ensemble de conditionnement et d'application 100 du mascara et un dispositif de chauffage 50, séparé de l'ensemble de conditionnement et d'application.
Les deux dispositifs 100 et 50 peuvent être vendus ensemble dans un même conditionnement, de type blister pack. L'unité 100 contenant le produit peut être vendue séparément.

L'ensemble de conditionnement et d'application 100 comprend un récipient 2, comprenant la seconde composition selon l'invention, surmonté d'un col fileté 3 dont un bord libre délimite une ouverture 4. Dans l'ouverture 4, est monté un organe d'essorage 5. L'ensemble 100 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient. De tels ensembles de conditionnement et d'application sont bien connus.
Le dispositif de chauffage 50 est conforme à ce qui est décrit dans le brevet US 6 009 884. Il comprend principalement une partie de préhension 51 et un capuchon 52. Une batterie est disposée à l'intérieur de la partie de préhension 51, et est connectée à un fil chauffant 53 configuré sous forme d'un enroulement hélicoïdal disposé sur une tige 54. Un « switch » 55 permet de mettre en tension, respectivement d'éteindre le dispositif. Une LED 56, lorsqu'elle change de couleur, indique que le dispositif est à la température requise, et qu'il est donc prêt à être utilisé.
L'alimentation de la partie chauffante via la batterie est en 12 V. La puissance dissipée est d'environ 1 Watt. Le fil chauffant 53 peut être réalisé en un alliage nickel/chrome.

Dans le mode de réalisation de la figure 2, l'applicateur 12 est constitué d'un cylindre métallique dont au moins une partie de sa périphérie, est striée perpendiculairement à son axe longitudinal. Le cylindre strié est fixé, notamment par collage, au bout de la tige 13. De manière diamétralement opposée relativement à la partie striée, est disposée une résistance chauffante 53, s'étendant sur sensiblement toute la longueur de l'applicateur 12. La résistance chauffante 53 peut être disposée dans une rainure ménagée longitudinalement dans la surface du cylindre.
Ainsi, la résistance chauffante 53 chauffe la seconde composition présente sur le cylindre strié, la zone striée de ce dernier servant à l'application proprement dit du produit sur les cils, ainsi qu'à leur séparation.

Selon un mode de réalisation, la seconde composition est appliqué à froid de manière classique sur les cils au moyen d'une brosse 12 puis chauffé après application : l'utilisatrice met en engagement la partie chauffante 53 du dispositif 50 avec les cils de manière à porter le dépôt de produit à la température de filant de la seconde composition puis étiré au moyen du dispositif chauffant formé sur les cils de manière à créer des fils dans le prolongement des cils. En refroidissant, les fils sont figés dans le prolongement des cils permettant l'obtention d'un effet allongeant.

Selon un autre mode de réalisation, le mascara est sous forme solide et est utilisé avec un dispositif de chauffage 50 seul. Il est mis en contact avec la partie chauffante 53 du dispositif 50 puis chauffé de manière à porter le dépôt de

produit à la température de filant de la seconde composition. Puis l'utilisatrice met en engagement la partie chauffante 53 du dispositif avec les cils puis étire au moyen du dispositif le dépôt formé sur les cils de manière à créer des fils dans le prolongement des cils.

Les exemples figurant ci-après sont présentés à titre illustratif et non limitatif de l'invention. Sauf indication contraire, les quantités indiquées sont exprimées en pourcentage massique.

**Exemple 1**

Premières Compositions

| | Exemple A | Exemple B (comparatif) | Exemple C (comparatif) | Exemple D (comparatif) |
|---|---|---|---|---|
| Copolymère acrylamide/AMPS dans isohexadecane[1] | 5 | 5 | 0 | 0 |
| PEG/PPG-17/18 Dimethicone[2] | 5 | 0 | 5 | 0 |
| Chlorure de sodium | 0.75 | 0.75 | 0.75 | 0.75 |
| Hydroxyethylcellulose | 2 | 2 | 2 | 2 |
| Eau | qsp 100% | qsp 100% | qsp 100% | qsp 100% |
| [1]Simulgel 600 de Seppic<br>[2]DC Q2-5220 Resin Modifier de Dow Corning | | | | |

Seconde composition

La seconde composition est réalisée par mélange à l'aide d'un extrudeur dont les fourreaux sont chauffés à 100°C :

Mélange de cire de paraffine et de copolymère éthylène/acétate de vinyle

(Coolbind de National Starch) 97%
Oxyde de fer noir 3%

Chaque première composition est utilisée pour maquiller trois éprouvettes de cils. Après un séchage de 2 minutes on applique la seconde composition préalablement chauffée à 60°C à l'extrémité des cils, en étirant le dépôt à l'aide d'un applicateur pour former des extensions de cils.

Chaque éprouvette est ensuite pincée dans un coton imprégné de lotion démaquillante EFFACIL de Lancôme, pendant 10 secondes puis on tire le coton.

On note ensuite le pourcentage d'extension de cils qui sont entièrement démaquillées. Cette opération est réalisée soit 5 minutes après la formation des extensions de cils, soit après 24 heures de séchage.

| | Exemple A | Exemple B (comparatif) | Exemple C (comparatif) | Exemple D (comparatif) |
|---|---|---|---|---|
| % cils démaquillés après 5 minutes | 100 | 70 | 70 | 0 |
| % cils démaquillés après 24 heures | 90 | 10 | 50 | 0 |

L'application de la composition A selon l'invention préalablement à la seconde composition permet de démaquiller entièrement les cils ce qui n'est pas le cas pour les exemples comparatifs B à D.

**Exemple 2**

Premières compositions

| | Composition E | Composition F | Composition G |
|---|---|---|---|
| Beurre de Jojoba | 6,3 | 6,3 | 6,3 |
| Huile de Jojoba | 6,3 | 6,3 | 6,3 |
| Cire de carnauba | 3,0 | 3,0 | 3,0 |
| Cire abeille | 5,5 | 5,5 | 5,5 |
| Stéareth 20 | 1,5 | 1,5 | 1,5 |
| Stéareth 2 | 1,2 | 1,2 | 1,2 |
| PEG-200 Glyceryl Stearate | 1,5 | 1,5 | 1,5 |
| Oxyde de fer | 7,1 | 7,1 | 7,1 |
| Ethanol | 3,0 | 3,0 | 3,0 |
| Propylène glycol | 3,0 | 3,0 | 3,0 |
| Copolymère acrylamide/AMPS dans isohexadecane[1] | 1,75 | 5 | 5 |
| Chlorure de Sodium | 0 | 0.75 | 0.75 |
| Hydroxyethylcellulose | 0,9 | 0,9 | 0,9 |
| Gomme arabique | 3,4 | 3,4 | 3,4 |
| PEG/PPG-17/18 Dimethicone[2] | 5,2 | - | 5,2 |
| PEG/PPG-16 Dimethicone / caprylic capric triglyceride[3] | - | 1,5 | - |
| Eau | Qs 100 | Qs 100 | Qs 100 |

[1]Simulgel 600 de Seppic
[2]DC Q2-5220 Resin Modifier de Dow Corning
[3]Abil Care 85 de Degussa

Seconde composition

La seconde composition est réalisée par mélange à l'aide d'un extrudeur dont les fourreaux sont chauffés à 100˚C :

| | |
|---|---|
| Mélange de cire de paraffine et de copolymère éthylène/acétate de vinyle | |
| (Coolbind de National Starch) | 97% |
| Oxyde de fer noir | 3% |

L'application sur les cils d'une des premières compositions E à G puis de la deuxième composition permet d'obtenir un maquillage des cils à la fois chargeant et très allongeant.
La tenue de ce maquillage est bonne et le démaquillage se fait facilement à l'aide d'un coton imbibé avec une lotion démaquillante de type Effacil de Lancôme.

**Exemple 3 : Composition comprenant une forte proportion d'agent filmogène et avec un démaquillage facile**

| | |
|---|---|
| Beurre de Jojoba | 6,3 |
| Huile de Jojoba | 6,3 |

(suite)

| | |
|---|---|
| Cire de carnauba | 3,0 |
| Cire abeille | 5,5 |
| Stéareth 20 | 1,5 |
| Stéareth 2 | 1,2 |
| PEG-200 Glyceryl Stearate | 1,5 |
| Oxyde de fer | 7,1 |
| Ethanol | 3,0 |
| Propylène glycol | 3,0 |
| Copolymère acrylamide/AMPS dans isohexadecane[1] | 1,75 |
| Chlorure de Sodium | 0 |
| Hydroxyethylcellulose | 0,9 |
| Gomme arabique | 3,4 |
| PEG/PPG-16 Dimethicone / caprylic capric triglyceride[2] | 2 |
| Copolymère acrylate d'éthyle/méthacrylate de méthyle (80/20) en dispersion aqueuse à 50% en matière sèche[3] | 20 |
| Eau | Qs 100 |

[1]Simulgel 600 de Seppic
[2]Abil Care 85 de Degussa
[3]Daitosol 5000 AD de Daito

La même composition sans copolymère acrylamide/AMPS dans isohexadecane et sans PEG/PPG-16 Dimethicone/caprylic capric triglyceride est difficile à démaquiller.

**Exemple 4 : Kit de composition avec une deuxième composition comprenant une forte proportion d'agent filmogène**

| | Première composition | Deuxième composition |
|---|---|---|
| Copolymère acrylamide/AMPS dansisohexadecane[1] | 5 | 5 |
| PEG/PPG-17/18 Dimethicone[2] | 5 | |
| Chlorure de sodium | 0.75 | - |
| Hydroxyethylcellulose | 2 | - |
| Copolymère Styrène-Acrylique en émulsion aqueuse à 40% en matière sèche[3] | - | 60 |
| Oxyde de Fer | - | 7 |
| Butylène Glycol | - | 4 |
| Ethanol | - | 3 |
| Eau | qsp 100% | qsp 100% |

[1]Simulgel 600 de Seppic
[2]DC Q2-5220 Resin Modifier de Dow Corning
[3]Syntran 5760 d'Interpolymer

Dans cet exemple la deuxième composition appliquée seule a un démaquillage très difficile en fin de journée, ce qui n'est pas le cas pour le kit.

**Exemple 5: Kit de composition avec une deuxième composition anhydre comprenant un polymère filmogène liposoluble**

La première composition est identique à celle de l'exemple 1 (composition A).

La deuxième composition est la suivante :

| | |
|---|---|
| Copolymère Ethylène/Vinyl acétate (Coolbind de National Starch) | 2,8 |
| Cire de Paraffine | 2.2 |
| Cire d'abeille synthétique (Kesterwax K82P de Koster Keunen ; nom INCI : synthetic beeswax) | 26 |
| Copolymère vinyle acétate/stéarate d'allyle (Mexomère PQ de Chimex) | 2,2 |
| Polylaurate de vinyle (Mexomère PP de Chimex) | 0,7 |
| Microbilles de cire de paraffine (Microease 114S de Micropowder) | 4 |
| Carbonate de propylène | 0,5 |
| Ethanol | 2,0 |
| Oxyde de fer | 4,6 |
| Silice | 10 |
| Bentone | 1,5 |
| Isododécane | Qs 100 |

Cette deuxième composition appliquée seule ne se démaquille pas à l'aide de démaquillant classique en phase aqueuse du type Effacil de Lancôme.
L'utilisation combinée de la première puis de la deuxième composition permet un démaquillage total.

**Revendications**

1. Kit de maquillage et/ou de soin des fibres kératiniques, notamment des cils ou des sourcils, comprenant :

   - une première composition cosmétique de maquillage ou de soin non thérapeutique des fibres kératiniques comprenant une phase aqueuse continue, au moins un tensioactif siliconé et au moins un polyélectrolyte réticulé ; et
   - une seconde composition de maquillage et/ou de soin des fibres kératiniques.

2. Kit selon la revendication 1, **caractérisé en ce que** la phase aqueuse de la première composition représente de 30% à 98% en poids, par rapport au poids total de ladite composition.

3. Kit selon la revendication 1 ou 2, **caractérisé en ce que** le tensioactif siliconé de la première composition est choisi parmi les agents tensioactifs siliconés non ioniques de HLB supérieur ou égal à 8 à 25°C, les tensioactifs siliconés amphotériques et leurs mélanges.

4. Kit selon l'une des revendications 1 à 3, **caractérisé en ce que** le tensioactif siliconé de la première composition est choisi parmi le polydiméthylsiloxane à terminaison oxyéthylène/oxypropylène en mélange avec des triglycérides d'acides caprylique/caprique (nom INCI : BIS-PEG/PPG-16/16 PEG/PPG-16/16 Dimethicone / Caprylic/Capric Triglyceride), le polydiméthylsiloxane à groupement alpha-omega polyéther (OE / OP: 40 / 60) (nom INCI : BIS-PEG/PPG-20/20 Dimethicone), le polydiméthylsiloxane oxyéthyléné oxypropyléné (nom INCI : PEG/PPG-20/6 Dimethicone) et le polydiméthyl / méthylsiloxane oxyéthyléné oxypropyléné (nom INCI : PEG/PPG-4/12 Dimethicone), la diméthicone copolyol (nom INCI : PEG/PPG-17/18 DIMETHICONE), la diméthicone copolyol benzoate, les diméthicone copolyols phosphates, et leurs mélanges.

**5.** Kit selon l'une des revendications précédentes, **caractérisé en ce que** le polyélectrolyte réticulé de la première composition est choisi parmi :

- le copolymère acrylamide / acide 2-méthyl 2-[(1-oxo 2-propényl)amino] 1-propanesulfonique (AMPS) partiellement ou totalement salifié ;
- le glycolate sodique d'amidon réticulé sous forme de poudre,
- les polyacrylates de sodium réticulés,
- les copolymères acide polyacryliques acrylates d'alkyle,
- l'AMPS,
- les copolymères AMPS/méthacrylates d'alkyle polyoxyéthylénés réticulés,
- leurs mélanges.

**6.** Kit selon l'une des revendications précédentes, **caractérisé en ce que** la seconde composition comprend au moins un polymère filmogène sous forme de particules en dispersion dans une phase aqueuse, ledit polymère étant présent en quantité au moins égale à 5% en matières sèches.

**7.** Kit selon l'une des revendications 1 à 5, **caractérisé en ce que** la seconde composition comprend au moins un composé ou un mélange de composés qui, lorsque la composition est portée à une température supérieure ou égale à 40˚C, confère à ladite composition un caractère filant dmax supérieur ou égal à 5 mm, et **en ce que** le kit comprend un dispositif pour l'application desdites compositions ; et/ou des moyens de chauffage pour porter, antérieurement simultanément ou postérieurement à son application, ladite seconde composition à une température supérieure ou égale à 40˚C.

**8.** Kit selon la revendication 7, **caractérisé en ce que** le composé ou le mélange de composés conférant à ladite seconde composition un caractère filant dmax supérieur ou égal à 5 mm est choisi parmi :

a) les polymères et copolymères comprenant au moins un monomère alcène, en particulier les copolymères à base d'éthylène,
b) les homopolymères polyacétates de vinyle,
c) les résines siliconées,
d) les polymères éthyléniques séquencés filmogènes, qui comprennent de préférence au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence,
e) les copolymères de diènes et de styrène,
f) les sulfopolyesters,
g) les cires,
h) les fibres, et leurs mélanges.

**9.** Kit selon l'une des revendications 7 ou 8, **caractérisé en ce que** le composé ou le mélange de composés conférant à ladite seconde composition un caractère filant dmax supérieur ou égal à 5 mm est choisi parmi :

- les copolymères d'alcène et d'acétate de vinyle, en particulier les copolymères d'éthylène et d'acétate de vinyle ;
- les copolymères d'éthylène et d'octène,
- les homopolymères polyacétates de vinyle,
- les résines de silicone T, telles que les polyphenylsiloxanes,
- les copolymères éthyléniques séquencés filmogènes est issus essentiellement de monomères choisis parmi les méthacrylates d'alkyle, les acrylates d'alkyle, et leurs mélanges,
- les copolymères de butadiène et de styrène,
- les copolymères obtenus par condensation de di-éthylèneglycol, cyclohexane di-méthanol, acide isophtalique, acide sulfoisophtalique, et leurs mélanges.

**10.** Kit selon l'une des revendications 7 à 9, **caractérisé en ce que** le composé ou le mélange de composés conférant à ladite seconde composition un caractère filant dmax supérieur ou égal à 5 mm est choisi parmi les mélanges de cire et de copolymère d'éthylène et d'acétate de vinyle.

**11.** Kit selon l'une des revendications 7 à 10, **caractérisée en ce que** le composé ou le mélange de composés conférant

à ladite seconde composition un caractère filant dmax supérieur ou égal à 5 mm est présent en une teneur en matières sèches allant de 5 à 100% en poids par rapport au poids total de la composition, de préférence allant de 10 à 100% en poids et mieux de 15 à 100% en poids.

**12.** Procédé cosmétique de maquillage ou de soin non thérapeutique des fibres kératiniques comprenant l'application sur les fibres kératiniques

- d'une première composition selon l'une des revendications 1 à 5, puis
- d'une seconde composition selon l'une des revendications 7 à 10, ladite seconde composition étant, antérieurement, simultanément, ou postérieurement à son application, portée à une température supérieure ou égale à 40°C.

**13.** Procédé selon la revendication 12, **caractérisé en ce que** la seconde composition est appliquée sur l'extrémité supérieure des fibres kératiniques, en particulier des cils.

**14.** Procédé selon l'une des revendications 12 ou 13, **caractérisé en ce que** la seconde composition est portée à une température supérieure ou égale à 45°C, mieux supérieure ou égale à 50°C et encore mieux, supérieure ou égale à 60 °C.

**15.** Composition comprenant une phase aqueuse continue, au moins un tensioactif siliconé non ionique de HLB supérieure ou égale à 8 à 25°C en une teneur allant de 0,2 à 20% en poids par rapport au poids total de ladite composition, et au moins un polyélectrolyte réticulé choisi parmi le copolymère acrylamide / acide 2-méthyl 2-[(1-oxo 2-propényl) amino] 1-propane sulfonate de sodium, le glycolate sodique d'amidon réticulé sous forme de poudre, les polyacrylates de sodium réticulés, les copolymères acide polyacryliques acrylates d'alkyle, les copolymères AMPS/méthacrylates d'alkyle polyoxyéthylénés réticulés, et leurs mélanges, et éventuellement au moins 5% en poids de matières sèches de polymère filmogène sous forme de particules en dispersion dans une phase aqueuse.

Fig. 1

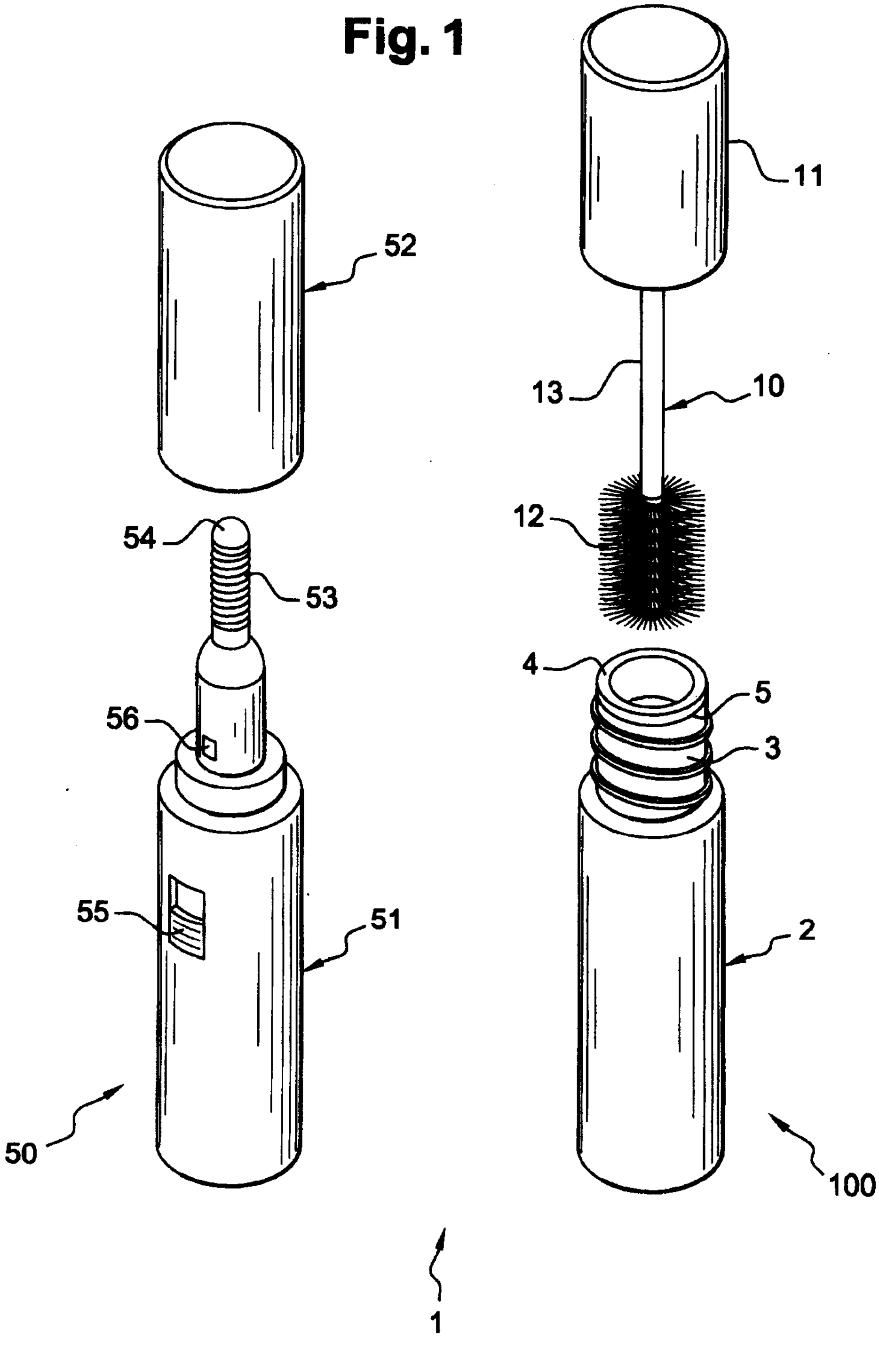

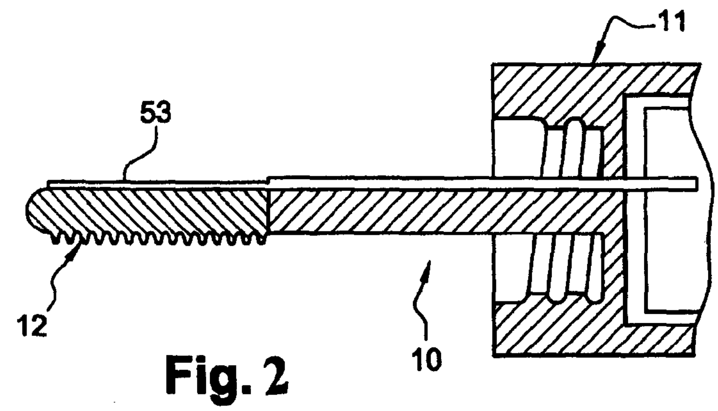


Fig. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2785801
- FR 2792190 A
- FR 2232303 A
- US 5162410 A
- WO 2004073626 A
- EP 1411069 A
- WO 04028488 A
- EP 847752 A
- US 4887622 A
- FR 2796529
- FR 2761959
- US 6009884 A
- US 5853010 A
- US 6220252 B


### Littérature non-brevet citée dans la description


- **GRIFFIN.** *J. Soc. Cosm. Chem.,* 1954, vol. 5, 249-256
- **KIRK-OTHMER.** Encyclopedia of Chemical Technology. WILEY, 1979, vol. 22, 333-432
- Handbook of Pressure Sensitive Adhesive. 1989, 609-619
- Polymer Handbook. John Wiley, 1989